# EUROPEAN PATENT SPECIFICATION

(11) **EP 3 824 372 B1**
(45) Date of publication and mention of the grant of the patent: **23.08.2023**
(21) Application number: 20707624.1
(22) Date of filing: 27.02.2020
(51) Int. Cl.: G06F 3/01, A61N 1/05, A61N 1/36, A61B 5/00, A61B 5/11, A61B 5/24, A61B 5/369

(54) **NEURONAL COMMUNICATION SYSTEM**
NEURONALES KOMMUNIKATIONSSYSTEM
SYSTÈME DE COMMUNICATION NEURONAL

(30) Priority: 27.02.2019 DE 102019202666; 24.06.2019 DE 102019209096; 26.09.2019 DE 102019214752
(43) Date of publication of application: 26.05.2021
(73) Proprietor: CereGate GmbH, 81671 München (DE)
(72) Inventor: VÁRKUTI, Bálint, 81679 München (DE)
(74) Representative: Bardehle Pagenberg Partnerschaft mbB Patentanwälte Rechtsanwälte
(86) International application number: PCT/EP2020/055156
(87) International publication number: WO 2020/174051

(56) References cited:
- WO-A1-2018/109715
- US-A1- 2009 306 741
- US-A1- 2010 094 382
- US-A1- 2010 094 382
- US-A1- 2017 182 328
- US-A1- 2017 182 328
- US-B2- 8 380 314
- ETHAN HEMING ET AL: "COMMUNICATION; Designing a somatosensory neural prosthesis: percepts evoked by different patterns of thalamic stimulation", JOURNAL OF NEURAL ENGINEERING, INSTITUTE OF PHYSICS PUBLISHING, BRISTOL, GB, vol. 7, no. 6, 1 December 2010 (2010-12-01), page 64001, XP020184271, ISSN: 1741-2552, DOI: 10.1088/1741-2560/7/6/064001
- HEMING E A ET AL: "Designing a Thalamic Somatosensory Neural Prosthesis: Consistency and Persistence of Percepts Evoked by Electrical Stimulation", IEEE TRANSACTIONS ON NEURAL SYSTEMS AND REHABILITATIONENGINEERING, IEEE SERVICE CENTER, NEW YORK, NY, US, vol. 19, no. 5, 1 October 2011 (2011-10-01), pages 477-482, XP011411577, ISSN: 1534-4320, DOI: 10.1109/TNSRE.2011.2152858
- ETHAN HEMING ET AL: "COMMUNICATION; Designing a somatosensory neural prosthesis: percepts evoked by different patterns of thalamic stimulation", JOURNAL OF NEURAL ENGINEERING, INSTITUTE OF PHYSICS PUBLISHING, BRISTOL, GB, vol. 7, no. 6, 1 December 2010 (2010-12-01), page 64001, XP020184271, ISSN: 1741-2552, DOI: 10.1088/1741-2560/7/6/064001
- ROELFSEMA PIETER R ET AL: "Mind Reading and Writing: The Future of Neurotechnology", TRENDS IN COGNITIVE SCIENCES, ELSEVIER SCIENCE, OXFORD, GB, vol. 22, no. 7, 2 May 2018 (2018-05-02), pages 598-610, XP085407742, ISSN: 1364-6613, DOI: 10.1016/J.TICS.2018.04.001

## Description

### 1. Technical Field

The present invention relates to signal and data processing systems for providing neuronal stimulation signals that may be used for direct neuronal communication with an individual such as for providing neuronal stimulation signals that may be used for behavior and, in particular, movement modification as well as for providing neuronal stimulation signals that may be used for signaling or communicating a device status to the individual.

### 2. Technical background

Communication with a human individual conventionally relies on providing sensory stimuli to the sensory organs of the individual, for instance, as a sequence of images, visual symbols, sounds and / or somatosensory stimuli. Examples for such information carrying sequence of sensory stimuli are: spoken language, script, Morse code, Braille, sign language, etc., wherein, for instance, the information content of spoken language and Morse code is communicated to the individual via auditory stimuli, the information content of Braille via somatosensory stimuli and the information content of script and sign language via visual stimuli.

To extract the communicated information, the sensory organs (e.g. the inner hair cells of the cochlea or the rod cells of the retina) transform the input energy of a respective sensory stimulus into neuronal excitation signals (i.e. into a sequence of action potentials). These neuronal excitation signals are then transmitted via afferent sensory nerve fibers to the brain and are ultimately processed by the cortex. The resulting mental re-creation of a given sensory stimulus in the cerebral cortex (i.e. the elicited cortical excitation pattern) is called the sensory percept associated with a given sensory stimulus. Sensory percepts can either be conscious or unconscious. For instance, a human trained in reading Braille with his fingertips does no longer consciously perceive the exact dot pattern of every Braille symbol of a Braille text but directly obtains a conscious understanding of the information content communicated via the sequence of Braille symbols forming the text.

Importantly, in many cases, the communicated information goes beyond the mere sensory percept. As an example, consider Morse code. Each Morse code message elicits a certain sequence of sensory precepts in the auditory cortex, i.e. a sequence of perceived tones having a certain duration (e.g. long or short) and having a certain pitch, (e.g. 500 Hz).

However, the cortex of an individual can also extract complex conceptual information from such sequence of sensory percepts of the cortex, for instance, extracting a request to provide help for the crew of a drowning ship encoded in a specific sequence of nine consecutive tones in Morse code. In the remainder of this application the term *"conceptual information*" is thus to be understand broadly, in the sense to comprise any information content that can be extracted by the cortex and goes beyond eliciting a mere sensory percept. Moreover, in many cases, the cortex acquires the capability to extract complex conceptual information from a given sequence of sensory percepts via learning. For instance, a radio operator first must learn the semantics of Morse code to understand an SOS signal.

Certain diseases and / or injuries of the sensory organs (e.g. deafness, blindness, etc.) may impair the communication capability of an individual. For some of these conditions, sensory stimulation devices such as cochlea or retina implants are well known in the prior art. Moreover, recent advances in data and signal processing as well as sensory stimulation technology have resulted in devices for eliciting visual percepts via stimulating somatosensory cells in the human tongue (see for example US 2006/0241718 A1, US 2009/0306741 A1and US 2015 / 0290453 A1). In principle, such devices can also be used for communication, e.g. for reading script or understanding general visual symbols.

In addition, considerable scientific research is directed to the development of so-called computer brain interfaces (CBIs) that allow to directly elicit certain sensory percepts in the cortex of an individual without stimulation of the sensory organs and / or the peripheral nervous system. For instance, the recent publication *"*Dynamic Electrical Stimulation of Sites in Visual Cortex Produces Form Vision in Sighted and Blind Humans"; M.S. Beauchamp et. al.; DOl. 10.1101/462697*,* describes how a subdural electrode array can be used to directly elicit visual percepts in the visual cortex of an individual allowing for immediate recognition of a variety of letter shapes without training and high accuracy. Similar methods and stimulation paradigms are described in "Engineering Artificial Somatosensation Through Cortical Stimulation in Humans"; B. Lee et. al.; Frontiers in Systems Neuroscience, 12; 2018; relating to eliciting somatosensory percepts via cortical stimulation with a subdural mini-electrocorticography grid. A general overview on trends in neuronal stimulation and measurement technology is provided by the article "Mind Reading and Writing: The Future of Neurotechnology"; P.R. Roelfsema et al.; Trends in Cognitive Sciences; 5; 2018*.*

A related research direction is discussed in "Sensory percepts induced by microwire array and DBS microstimulation in human sensory thalamus"; B.D. Swan et. al.; Brain Stimulation; 11; 2018*.* Specifically, the authors could show that it is possible to use deep brain stimulation (DBS) electrodes to evoke somatosensory percepts of the hand or arm by electrostimulation of the sensory thalamus in humans.

Further, the publication "Optimized programming algorithm for cylindrical and directional deep brain stimulation electrodes"; D.N. Anderson et. al.; Journal of Neural Engineering; 15; 2018*;* relates to using magnetic resonance imaging (MRI) and diffusion tensor imaging (DTI) to build a finite element method (FEM) model of a DBS electrode used for treatment of movement and psychiatric disorders (e.g. morbus Parkinson) and of the brain tissue surrounding the DBS electrode. The resulting FEM model can then be used to find optimized stimulation parameters for stimulating efferent cortico-motor axons via the DBS electrode.

Further, WO 2016/116397 A1 relates to a medical data processing method for determining an orientation of nerve fibers relative to a non-physiological electric field, produced by a stimulation electrode based on medical image data of nerve tissue comprising white matter nerve fibers. Additional prior art that may be relevant is provide by KR 10 184 1625 und EP 3 431 138.

Further, WO 2018/109715 A1 relates to a brain computer interface system for modulating cognitive performance. A processor is programmed to process signals sensed in the one or more cortical regions for detecting an indication associated with an intention to perform a cognitive task, the presentation of a cognitive task and/or the performing of a cognitive task, and to control the stimulating of the one or more target brain regions responsive to the detecting of the indication for modulating the cognitive performance of the user. The system processes the sensed signals for detecting a neuronal activity pattern that is an indication of an intention of the user to perform a cognitive task and/or associated with the performance of a cognitive task by the user.

Further, US 8,380,314 B2 relates to an implantable medical device (IMD) comprising a biosignal detection module that monitors an EEG signal from within the brain of a patient and determines whether the EEG signal includes the biosignal. The biosignal detection module analyzes frequency components of the EEG signal. In this manner, the patient may adjust therapy delivery by providing a volitional input that is detected by brain signals. Example therapies include electrical stimulation, drug delivery, and delivery of sensory cues. For instance, the IMD may deliver electrical stimulation to a sensory location within brain to cause the perception of an external stimulus. For example, the IMD may deliver stimulation to a visual cortex of the brain in order to simulate a visual cue.

In addition, the article HEMING E A ET AL: "Designing a Thalamic Somatosensory Neural Prosthesis: Consistency and Persistence of Percepts Evoked by Electrical Stimulation", IEEE TRANSACTIONS ON NEURAL SYSTEMS AND REHABILITATIONbENGINEERING, vol. 19, no. 5, 1 October 2011, discusses neurostimulation experiments performed on five subjects implanted with DBS electrodes for treatment of essential tremor and neuropathic pain. Stimulation signals are generated by a constant voltage implantable pulse generator controlled by telemetry using the N'Vision Clinician Programmer (Model 8840, Medtronic Inc., Minneapolis, MN). The study protocol involved bipolar stimulation applied through each patient's thalamic DBS electrode (Model 3387, Medtronic Inc., Minneapolis, MN) Stimulating electrode pair, voltage, frequency, and duty cycle were varied manually within the limits of the programmer.

Similarly, the article HEMING E. et al: "Designing a somatosensory neural prosthesis: percepts evoked by different patterns of thalamic stimulation"; JOURNAL of NEURAL ENGINEERING 7 (2010) 064001 (7pp) discusses a clinical study that explored whether a thalamic site for a somatosensory neural prosthetic could provide natural somatic sensation to humans. Different patterns of electrical stimulation (obtained from thalamic spike trains) were applied in patients undergoing deep brain stimulation surgery. Changes in pattern produced different sensations, while preserving somatotopic representation. While most percepts were reported as unnatural, some stimulations produced more natural sensations than others. However, the additional patterns did not elicit more natural percepts than high-frequency (333 Hz) electrical stimulation. The authors conclude that these features suggest that despite some limitations, the thalamus may be a feasible site for a somatosensory neural prosthesis and different stimulation patterns may be useful in its development.

US 2017/182328 A1 relates to a neurostimulation system configured for providing neuro stimulation therapy to a patient. A user customizes tonic and non-tonic pulse patterns on a pulse-by-pulse basis. The patterns can be stored and selected by the user.

Electrical stimulation energy is delivered to at least one electrode in accordance with the customized pulse pattern. The stimulation can be used to deliver unique information to the nervous system using action potentials.

US 2017/182328 Aialso discloses an external control device that comprises memory configured for storing a plurality of different customized pulse patterns, in which case, the control circuitry may be configured for selecting one or more of the customized pulse patterns in response to an input from a user.

Further, US 2010/0094382 A1 relates to visual prosthesis having one or more electrodes operative to deliver electrical signals to a lateral geniculate nucleus of a mammal, a power supply operative to provide power to the electrodes, a visual information translator operatively connected to the electrode array, and a visual sensor operatively connected to the visual information translator. The visual prosthesis is operative to translate visual information into an electrical signal and transmit the electrical signal to electrodes to stimulate brain activity to recognize visual information.

However, the neuronal stimulation paradigms and systems known from the prior art have various deficiencies. First, CBIs that employ direct cortical stimulation, e.g. via a subdural micro-electrode array, can only stimulate the outer layers of the cortex and therefore neglect or at least do not fully respect the hierarchical organization of cortical processing. This results in many cases in non-physiological stimulation and / or excitations patterns potentially degrading the quality of the elicited sensory percepts. In other words, the neurons in the outer layer of the cortex are adapted to receive neuronal signals that have been pre-processed by various lower cortical layers and / or deeper brain regions and thus are in general not adapted to directly receive and process electric stimulation signals provided by a subdural electrode array.

Moreover, the spatial resolution of current micro-electrode arrays is still far too coarse as to be able to elicit physiological correct sensory percepts. In addition, it is well known in the art that the neuron and blood vessel rich environment of the cortex can easily lead to scar tissue formation and / or gliosis resulting in a reduced performance of the micro-electrode array over time.

Furthermore, micro-fabricated implants with single-unit / single-neuron interface capabilities or electro-cortical grids are usually anchored between the brain and the skull. Since the brain moves with every pulsation of the heart inside the cranial cavity, or during expose to acceleration forces e.g. during physical activity, the relative position of the surface implants can move over time - leading to a further degradation of the CBI.

Concerning the stimulation of sensory percepts via DBS of the thalamus, the methods and systems described in the prior art completely lack sufficient spatial and / or sensory resolution and thus can by no means be used for robust and reproducible stimulation of desired sensory percepts. Furthermore, they only aim at reproducing the natural sense of sensation in the human somatosensory system e.g. for application as a neural prosthetic for amputees.

In some aspects, the present application is directed to neuronal stimulation systems for behavior and movement modification, in particular, in the context of the treatment of neurological movement impairments.

Neurological diseases such as Parkinson's disease (PD), essential tremor or dystonia may severely degrade the movement and coordination abilities of affected patients. It is well known, that certain symptoms of such diseases can be successfully treated or at least ameliorated via stimulation of the nervous system of the affected patients.

For instance, deep brain stimulation (DBS) systems send electrical impulses, through implanted electrodes, to specific areas / nuclei of the brain to treat such symptoms. Conventionally, in the treatment of PD symptoms, these nuclei may include the globus pallidus interna, the thalamus and / or the subthalamic nucleus. It is known that DBS of the globus pallidus interna improves motor function while DBS of the thalamus improves tremor but has little effect on bradykinesia or rigidity. Further, DBS of the subthalamic nucleus is associated with reduction in PD medication.

US 2007/0250134 A1 relates to an implantable medical device for delivering different electrical stimulation therapies to the nervous system of a patient in order to suppress different symptoms of PD. One such electrical stimulation therapy is configured to suppress the so called *freezing of gait* (FOG) symptom while another such electrical stimulation therapy is configured to suppress other PD symptoms such as tremor, bradykinesia or rigidity. At any given time, the medical device delivers the electrical stimulation therapy according to a current set of therapy parameters. The therapy parameters may change over time. The medical device, or another device, periodically determines an activity level of the patient, and associates each determined activity level with the current therapy parameter set. In addition to recording FOG events and determining activity metric values based on such events, the medical device may also control delivery of a stimulus to terminate FOG. For example, if stimulation leads are implanted proximate to the spinal cord or peripheral nerves of the patient the medical device may control delivery of a stimulation perceivable by the patient to prompt the patient to walk, thereby terminating FOG. The stimulation may be rhythmic, e.g., may approximate the rhythm of walking, which may prompt the patient to walk and thereby terminate the FOG.

The recent publication "Sensory Electrical Stimulation Cueing May Reduce Freezing of Gait Episodes in Parkinson's Disease"; L. Rosenthal et. al.; Hindawi Journal of Healthcare Engineering; 2018, Article ID 4684925 describes how skin surface electrodes can be used to provide a fixed rhythmic sensory electrical stimulation signal to PD patients in order to reduce the time taken to complete a walking task and to reduce the number of FOG episodes occurring when performing the task.

A different approach for treatment of movement impairments consists in rhythmic auditory cueing. The recent review article "Effect of rhythmic auditory cueing on parkinsonian gait: A systematic review and meta-analysis"; S. Ghai et al.; NATURE SCIENTIC REPORTS; (2018) 8:506; DOI:10.1038/s41598-017-16232-5 provides a systematic overview on using rhythmic auditory cueing to enhance gait performance in PD patients.

Moreover, US 2019/0030338 A1 relates to an implantable medical device that is capable of determining whether a patient is susceptible to FOG events during ambulatory movement without the patient actually demonstrating an episode of FOG. The implantable medical device senses, via one or more electrodes, a bioelectrical signal of a brain of the patient while the patient performs a movement associated with FOG. The implantable medical device then determines, based on the sensed bioelectrical signal, whether the patient is susceptible to FOG while the patient is not experiencing an episode of FOG. Further, upon detecting the movement associated with FOG, the implantable medical device delivers an electrical stimulation therapy via a DBS electrode to the patient configured to suppress FOG.

However, the electrical stimulation systems known from the prior art have various deficiencies. For instance, auditory cueing treatment for patients suffering from a movement impairment may degrade the listening capabilities of the patient and distract him from other relevant sounds providing crucial information on his environment.

Further, providing electrical stimulation signals via skin surface electrodes requires bulky electronic equipment to be carried by the patient as well as continuous maintenance of the skin surface electrodes that may degrade and /or detach from the skin due to external moisture or body moisture.

Moreover, conventional DBS systems can only be used to provide unspecific neuromodulation signals that for instance are configured to suppress FOG events or tremor. However, such systems completely lack the capability of continuously enhancing the movement of the patient after a FOG event has been suppressed in terms of regularity, balance and / or body posture.

In addition, similar to cardiac pacemakers, symptoms of neurological diseases such as Parkinson's disease (PD), essential tremor and dystonia are routinely treated via stimulation of the nervous system of the affected patients. For instance, deep brain stimulation (DBS) systems send electrical impulses through implanted electrodes to specific areas / nuclei of the brain to treat such symptoms. In the treatment of PD symptoms, these nuclei may include the globus pallidus interna, the thalamus and / or the subthalamic nucleus. It is known that DBS of the globus pallidus interna for instance improves motor function while DBS of the thalamus reduces tremor. Further, DBS of the subthalamic nucleus is associated with reduction in conventional PD medication.

Similar to the treatment of PD symptoms, electric stimulation devices can also be used for cardiac rhythm management, for treatment of Alzheimer's disease, dementia or depression as well as for communication purposes and implementations of computer-brain interfaces.

For instance, US 8,352,029 B2 relates to a method for implementing a neural stimulation therapy mode in an implantable medical device comprising the steps of mapping respective device states, defined by a neural event timer or indications of sensed physiologic events, to associated device actions in a stored neural table, storing an event represented as a device status word and a time stamp in a queue in response to an action input, and comparing current timer states or the indications of sensed physiologic events to a device state in the neural table and, if found to match, causing performance of associated device actions, wherein the device actions may neural stimulation energy delivery and / or a change in the timer states.

Similarly, US 7,751,884 relates to an implantable medical device comprising stimulation circuitry adapted to provide neural stimulation energy to a neural stimulation electrode, one or more timers, including at least one neural event timer, a device behavior memory including a neural table, and a comparison circuit. The neural table maps a particular device state defined at least in part by a neural event timer to one or more associated device actions that include a neural stimulation energy delivery and / or a change in state of at least one neural event timer. The comparison circuit is adapted to compare a current state of the timers to a device state in the neural table and, if found to match, causing performance of one or more associated device actions.

Further, US 8,812,128 relates to a rechargeable implantable medical device having a power source and being configured for monitoring the power source, generating a battery status signal based on the monitoring and configured for transcutaneously transmitting a communication signal and the battery status signal to an external device. The external device is configured for receiving the communication signal and the battery status signal from the rechargeable implantable medical device, changing from a low energy consumption state to a high energy consumption state in response to the received communication signal, and for generating a user-discernible signal in response to the received status signal.

Moreover, US 8,193,766 relates to a system for estimating a time to recharge a rechargeable power source of an implantable medical device. A plurality of measured parameters relating to the implantable medical device and an external charging device are applied to a model of recharging performance and an estimate is provided to a patient, in advance of charging. Once charging has begun, updated estimates can be provided until charging is complete. Once charging is complete, the model may be updated to reflect any differences in the estimated time to complete charging and the actual time required to complete charging. The model may be based on limitations to the rate at which charge may be transferred to the rechargeable power source over a plurality of intervals.

However, typically, the stimulation parameters employed by such medical stimulation devices are selected or configured such that the treated patient does not directly perceive whether his device is properly operating or not. For instance, devices such as implanted electric stimulation devices may be powered by a limited power source such as a battery that may gradually discharge over time. At some point the charging level of the battery may be insufficient for the stimulation device to work properly.

Similarly, such devices may also stop working due to hardware failure (e.g. caused by strong external magnetic fields), or physiological changes of the treated brain areas that receive the stimulation treatment.

In many cases, however, the patient does not or at least not immediately realize that his therapy device is no longer operating properly, in particular, if the device does not comprise a configuration or status interface that can be accessed by the patient himself but only by trained medical personal. Even if the device comprises such an interface that is accessible by the patient (e.g. a wireless remote control or telemetry device) there might be situations (e.g. long holidays, an empty battery of the remote control etc.) when the patient cannot access the interface and obtain device status information.

In such situations, the neurological symptoms normally treated by the respective therapy device may gradually worsen, which may also remain undetected by the patient for a prolonged period of time. This may be particularly problematic if the neurostimulation device is used for treatment of psychological conditions such as dementia, Alzheimer's disease, schizophrenia, bipolar disorder and / or depression. In such cases, a deactivation of the therapy device (e.g. caused by a low-battery or a false emergency deactivation caused by an external magnetic field) has no immediately perceivable effect and the patient will eventually only notice the undesired deactivation of his therapy device after a long time period when the untreated symptoms have already deteriorated substantially.

It is thus the problem of the present invention to provide novel neuronal stimulation systems that improve the known systems such that the above outlined disadvantages of the prior art are at least partially overcome.

### 3. Summary of the Invention

The above-mentioned problem is at least partly solved by the subject matter of the independent claims of the present application. Exemplary embodiments of the invention are the subject of the depended claims.

In one example, that is not part of the appended claims, the present invention provides a system for providing neuronal stimulation signals configured to elicit a sensory percept in the cortex of an individual, comprising: means for obtaining spatial information relating to the actual or planned position of at least one neuronal stimulation means relative to at least one afferent axon targeting at least one sensory neuron in the cortex of the individual and means for determining at least one neuronal stimulation signal to be applied to at least one afferent axon via the at least one neuronal stimulation means based at least in part on the obtained spatial information.

For instance, such systems are capable of determining specific neuronal stimulation signals that are tailored to stimulate a desired sub-population of sensory neurons in the cortex. For example, some neuronal stimulation signals may be engineered such that they are configured to elicit somatosensory percepts of the left hand of the individual, while other stimulation signal are configured to elicit a touch sensation of the tongue. By using the obtained spatial information of the electrode position relative to afferent axons targeting the sensory cortex the system can establish a correspondence between the actual form of the neuronal stimulation signal and a target region and / or a target percept in the sensory cortex. For instance, such neuronal stimulation signal may comprise a sequence of current pulses characterized by a pulse width, a pulse frequency, a pulse amplitude and / or a pulse shape.

In this manner, the provided system facilitates directly eliciting certain sensory percepts in the cortex of an individual without stimulation of the sensory organs and / or the peripheral nervous system.

Further, such neuronal stimulation signals may be configured to elicit a sequence of action potentials in the at least one afferent axon targeting the sensory neurons in the cortex of the individual. Moreover, the at least one axon may be a thalamocortical axon, i.e. an axon that transmits sensory information from the thalamus to the sensory cortex of an individual.

By providing neuronal stimulation signals that are configured to elicit action potentials in axons targeting the sensory cortex, it can be ensured that the stimulation for a given sensory percept enters the cortex via the physiological correct signaling pathway, e.g. via action potentials of a thalamocortical axon. In this manner, correct cortical processing of the neuronal stimulation signals can be ensured and the quality of the elicited sensory percept can be enhanced.

Further, the means for obtaining the spatial information may comprise means for obtaining tractography information and / or neuronal connectivity information for the at least one afferent axon, preferably comprising magnetic resonance imaging data, diffusion tensor imaging data and / or anatomic reference data.

By using such tractography information and / or neuronal connectivity information (e.g. information on synaptic connections between axons and sensory neurons) for the axons that target the sensory cortex of the individual the actual form of the neuronal stimulation signals can be even further be tailored to elicit a desired sensory percept in a desired region of the sensory cortex. In particular, DTI data may allow to also take in to account individual neuroanatomical variations and / or neuronal plasticity for determining the desired neuronal stimulation signals for a given target percept and / or target region of the sensory cortex.

Further, the means for obtaining the spatial information may comprise means for obtaining neuroimaging data of a volume of brain tissue surrounding at least a portion of the actual or planned position of the at least one neuronal stimulation means, preferably comprising computer tomography data and / or magnetic resonance imaging data.

In this way, the system can also take into account the physical and in particular electrical properties of the brain tissue surrounding the neural stimulation means. For instance, the neuroimaging data may be used to determine the electric conductivity of the surrounding brain tissue. In this way, the specificity and accuracy of the determined neuronal stimulation signal for certain afferent axons and / or sensory neurons can further be enhanced.

Further, the means for determining the neuronal stimulation signal may comprise means for determining an excitation probability of the at least one afferent axon and / or the at least one sensory neuron based at least in part on the obtained spatial information, preferably by using a finite element method and / or a neuronal compartment model.

In this manner active electric properties (e.g. the non-linear neuronal excitability) of the at least one axon can be taken into account by the system when determining the at least one neuronal stimulation signal, thereby further enhancing the specificity and accuracy of the neuronal stimulation signal for a desired target axon and /or target stimulation region in the sensory cortex.

The means for determining the at least one neuronal stimulation signal may be further configured to determine the at least one neuronal stimulation signal based at least in part on at least one of: at least one desired type of percept to be elicited by the at least one neuronal stimulation signal; at least one desired target area of the cortex comprising the at least at least one targeted sensory neuron; an optimization procedure maximizing the number of different sensory percepts that can be perceived by the individual when the determined at least one neuronal stimulation signal is applied to the at least one afferent axon via the at least one neuronal stimulation means.

In this manner, the amount of information that can be communicated via eliciting sensory percepts by a given stimulation means can be enhanced. Furthermore, by interfacing with axonal structures at points deep within the brain via dynamically configured electrical pulses information can be transmitted to the full range of superficial cortical processing zones these axons project to - without having to cover the entire surface area with invasive macro- or microstimulation implants.

In another example, the present disclosure provides a system for stimulating sensory neurons in a cortex of an individual, comprising: means for storing relations between sensory percepts of the individual and corresponding neuronal stimulation signals to be applied to at least one afferent axon targeting the sensory neurons of the individual and means for selecting and transmitting at least one of the neuronal stimulation signals to at least one neuronal stimulation means of the individual.

This embodiment greatly improves the efficiency and flexibility of eliciting desired sensory percepts in the cortex of an individual. For instance, a communication device that interfaces with the provided system can easily determine and directly transmit the specific neuronal stimulation signal corresponding to a desired sensory percept to be elicited in the cortex of an individual via stimulation of afferent axons targeting sensory neurons in the cortex of the individual.

For instance, in some embodiment of the present invention, the stored relations between the sensory percepts and the corresponding neuronal stimulation signals are based at least in part on one or more of: spatial information for the at least one afferent axon, spatial information for the at least one neuronal stimulation means, neuronal connectivity information for the at least one afferent axon, an electric field distribution associated with the at least one neuronal stimulation means, functional neuroimaging data for the individual, diffusion tensor imaging data for the individual, neuroanatomical reference data being relevant for the individual, cortical excitation data for the individual, perceptual and / or conceptual learning data for the individual, sensory perception data for the individual, behavioral data based at least in part on subjective experiences of the individual, an optimization procedure for maximizing the number of sensory percepts that can be perceived, preferably simultaneously, by the individual when the corresponding neuronal stimulation signal is transmitted to the at least one neuronal stimulation means of the individual.

In some embodiments, the provided system may further comprise at least one neuronal stimulation means adapted to induce a sequence of action potentials in the at least one afferent axon corresponding to the at least one neuronal stimulation signal.

Via integrating the neural stimulation means into the provided system the overall system efficiency can be enhanced and system complexity be reduced, e.g. by using customized wired or wireless interfaces for the at least one neuronal stimulation means.

To further improve the versatility and the degree of system integration, in some embodiments, the means for transmitting the at least one neuronal stimulation signal may comprise at least one of: a digital signal processor; a digital to analog converter; a radio frequency transmitter; a radio frequency receiver; an analog and / or digital signal amplifier; radio frequency mixing circuitry; low-pass, high-pass and / or bandpass circuitry; wireless communication circuitry; and impedance matching circuitry.

In some embodiments, the perceptual and / or conceptual learning data on which the relations between sensory percept and corresponding neuronal stimulation signal may be generated based at least in part on one or more of: the individual participating in a perceptional and / or conceptual learning procedure and the individual being analyzed by a functional neuroimaging device, preferably functional magnetic resonance imaging while receiving the at least one neuronal stimulation signal.

In this manner, the ability of the human brain to learn how to distinguish even fine variations in sensory percepts can be used to improve the system performance. The finer the resolution of the unique perceptual patterns the more concepts or messages can be uniquely associated with the neural stimulation patterns, similar to the relationship between a sign and its meaning. The more signs can be perceived the more meaning can be communicated.

Specifically, the present invention provides a system for communicating conceptual information to an individual as defined in independent claim 1**.** Specifically, according to claim 1, the stored relations link each sensory percept, elicited by the corresponding neuronal stimulation signal with the corresponding conceptual information. Further, according to claim 1, the stored relations are based at least in part on conceptual learning data for the individual, the conceptual learning data associating the plurality of conceptual information with the plurality of corresponding neuronal stimulation signals and the corresponding sensory percepts.

For instance, the conceptual information may comprise at least one of: a letter, a number, a color, a direction in space, a word, a sentence, an object, an identity of a person or animal and / or an instruction for a motor response of the individual, a position, a bio- or neurofeedback signal, a shape, an image or icon, a warning, an association, a degree of similarity, a salience signal, a rhythm, start or stop commands or information, touch information, surface texture information, pressure information, electromagnetic field strength indication or other types of information.

Further, according to claim 1, the means for selecting the at least one neuronal stimulation signal comprise means for accessing a data storing means storing relations, specific for the individual, between a plurality of conceptual information and a plurality of corresponding neuronal stimulation signals.

For instance, if an individual has participated in a perceptual and / or conceptual learning procedure and has learned to identify a certain somatosensory percept (e.g. a touch sensation on the left palm) with a piece of conceptual information (e.g. a direction in space) a relation between the conceptual information and the specific neuronal stimulation signal eliciting the respective somatosensory percept can be stored in a data storing means and be accessed for subsequent communication sessions with the individual.

For instance, the at least one neuronal stimulation signal may be adapted to evoke a conscious or an unconscious sensory percept in the cortex of the individual.

Depending on the actual application and / or technology platform used for stimulation the at least on afferent axon targeting the sensory cortex of the individual, the at least one neuronal stimulation means may comprise an electric-neuronal interface means, an opto-neuronal interface means and / or a chemical-neuronal interface means.

For instance, the electric-neuronal interface means may comprise at least one stimulation electrode, preferably comprising a plurality of independently controllable electric stimulation contacts. For example, such stimulation electrode may be a multi-contact DBS electrode adapted for targeting the thalamus region of the human brain.

Given a suitable signal source such multi-contact electrodes allow for independent multi-channel current control enabling to create complex spatial electric stimulation pattern around the stimulation electrode. In this way a single electrode can be used to selectively stimulate a desired subset of axons in the vicinity of the stimulation electrode. Essentially, the number of different axons or different axon bundles that can selectively be stimulated via such multi-channel electrode correspond to different neuronal communication channels that can be established via said electrode.

To further improve the spatial resolution of the excitation pattern that can be generated, the electric-neuronal interface means may further comprise at least two independently controllable stimulation electrodes, each preferably comprising a plurality of independently controllable electric contacts.

For example, the at least one stimulation electrode may be provided by at least a part of a neuromodulation electrode implanted for a therapeutic purpose independent from the stimulation. Specifically, the portion of the neuromodulation electrode may be a portion that is not used for the therapeutic purpose.

For instance, in many cases, a DBS electrode that is used as a neuromodulator, e.g. for treatment of Parkinson, is not always active and / or may comprise independently controllable contacts that are not required for achieving the therapeutic purpose. Thus, the neuromodulation electrode can also be used for applying neuronal stimulation signals provided by a system according to the present invention.

In general, the systems provided by the present invention may provide neuronal stimulation signals for the at least one afferent axon targeting the least one sensory neuron, which may be located in at least one of: a somatosensory cortex area, an auditory cortex area, a visual cortex area, an olfactory cortex area, a gustatory cortex area, a somatosensory association cortex area, and a proprioception cortex area.

In another example, not being part of the appended claims, the present invention provides a system for determining an implantation position or trajectory for a neuronal stimulation electrode, comprising: means for automatically identifying a plurality of afferent axons targeting sensory neurons in the cortex associated with a specific sensory modality; means for obtaining neuroanatomical imaging and / or reference data of a volume of brain tissue comprising the plurality of afferent axons and means for automatically determining the implantation position and / or implantation trajectory for the neuronal stimulation electrode based at least in part on the identified plurality of afferent axons and the obtained neuroanatomical imaging and / or reference data.

Specifically, the means for obtaining the neuroanatomical imaging data may comprise means for obtaining computer tomography and / or magnetic resonance imaging data.

For instance, the provided system allows to provide a neurosurgeon and / or a surgical robot with information on a desired implantation position and / or implantation trajectory for the neuronal stimulation electrode prior to performing the implantation of the electrode. For example, if the electrode is to be implanted for the purpose of establishing a CBI with the individual, the implantation position and / or implantation trajectory can be tailored for a specific sensory percept that is to be used for communication purposes, e.g. used to encode conceptual information to be communicated.

In some examples, the means for automatically determining the implantation position and / or the implantation trajectory may comprise means for optimizing a stimulation rate of at least a subset of the sensory neurons. In this way the communication bandwidth of a desired perceptual communication channel may be enhanced even prior to implantation of the electrode.

Further, the means for automatically determining the implantation position and / or the implantation trajectory may be configured to determine the implantation position and / or the implantation trajectory based at least in part on identifying at least two types of sensory percepts to be elicited by the neuronal stimulation electrode, preferably simultaneously.

In this manner the neurosurgeon and /or surgical robot is enabled to perform the implantation procedure such that at least two separate communication channels can be established for the CBI using the implanted electrode.

In essence, the present invention incorporates new methods for calibrating a neural interface optimally to the individual and explicitly builds on the brains natural ability of decoding any information-carrying signal of behavioral relevance into a subjectively interpretable perception or translate it into the appropriate corresponding action.

In another embodiment, not being part of the appended claims, the present invention provides a system for stimulating the sensory cortex of an individual, comprising: means for obtaining a neuronal stimulation signal adapted to provide a movement cue for the individual and means for transmitting the neuronal stimulation signal to an electric contact of a neuronal stimulation electrode that is implanted into the brain of the individual.

For example, the neuronal stimulation electrode may already be implanted into the brain of the individual for a purpose different from providing the movement cue.

In this manner, no additional electrode must be implanted but an existing one can be used for interfacing the system provided by the present invention.

For instance, the neuronal stimulation signal may be adapted to elicit a sensory percept, preferably conscious, in the cortex of the individual. The sensory percept may for example be elicited in in at least on of: a somatosensory cortex area; a visual cortex area and an auditory cortex area. By using such a system, sensory percepts can directly be elicited in the cortex of an individual without stimulation of the sensory organs and / or the peripheral nervous system.

For instance, the system provided by the present invention can be interfaced with a DBS electrode that is already implanted into the brain of an individual for the purpose to stimulate the thalamus or the sub-thalamic nucleus with a neuromodulation signal (e.g. for treatment of PD symptoms such as tremor). In this way, the provided system can provide the movement cue via stimulating afferent sensory axons that run in the vicinity of the thalamus and project into the sensory cortex of the brain. Such a system thus allows to provide various types of movement cues directly to the cortex without requiring additional sensory stimulation equipment such as earphones, skin surface contacts, dedicated neuronal stimulation electrodes etc. but makes use of electric contacts that are already available in the vicinity of such afferent sensory axons.

In other words, patients that have already been implanted with a neuronal stimulation electrode for a different purpose can easily also be provided with movement cues via interfacing their already present implant with the neuronal stimulation systems provided by the present invention without undergoing additional surgical procedures or requiring to carry additional equipment.

In some embodiments, the neuronal stimulation electrode may be implanted for the purpose of at least one of: deep brain stimulation; neuronal sensing; an open-loop or closed-loop combination of deep brain stimulation and neuronal sensing; treatment of Parkinson's disease, of epilepsy, dystonia and / or of tremor as well as neuronal communication.

Further, in some embodiments, the electric contact to which the neuronal stimulation signal is transmitted to may not be used for the purpose that is different from providing the movement cue. For instance, if a multi-contact DBS electrode is used for the purpose of applying a neuromodulation therapy such as a treatment of PD symptoms typically only a subset of its electric contacts (e.g. one contact) is actually used for applying the neuromodulation therapy stimulation signal. The remaining unused contacts can thus be used to stimulate afferent sensory axons targeting the sensory cortex of the individual and thereby to provide a sensory movement cue or other movement information to the patient.

Alternatively, an electric contact that is used for applying the neuromodulation therapy can also be used in an alternating manner. For instance, the movement cue may be provided during periods wherein the electrode is not used for applying the neuromodulation therapy (e.g. the purpose that is different from providing the movement cue).

Further, in some embodiments, the stimulation system provided by the present invention may also comprise means for operating the neuronal stimulation electrode according to its purpose. For instance, if the neuronal stimulation signal providing the movement cue is applied via an electric contact of a DBS electrode implanted for treatment of PD, the stimulation system provided by the present invention may also comprise the necessary means to generate, amplify and / or apply the neuromodulation therapy signal via the DBS electrode.

In this manner, system components such as a power supply, communication interfaces, memory, signal processing circuitry, etc. can be shared and be integrated into a single neurostimulation device providing both, the neuromodulation therapy signal and the neuronal stimulation signal that is adapted to provide the movement cue. This reduces, cost, complexity and power consumption of the combined stimulation system compared to using largely independent stimulation systems for each purpose alone.

Further, in some embodiments, the neuronal stimulation signal may comprise a signal or a pulse train signal designed to be perceived by the individual as periodic.

For instance, the neuronal stimulation signal may be designed such that it elicits periodically appearing sensory percept in the cortex of the individual. For example, the neuronal stimulation signal may elicit a periodically appearing pressure sensation of a body part such as a leg, a foot, a hand, a tongue etc. of the patient. Alternatively, or additionally, auditory and /or visual sensory percepts may be elicited in a periodic manner.

For instance, such a signal designed to be perceived by the individual as periodic may comprise burst pulses, wherein each burst may comprise a series of signal spikes. In this case, the perceived periodicity of such a signal may then correspond to the repetition rate of the bursts pulses. For instance, a burst pulse may be 300ms long and may comprise 42 signal spikes each having an amplitude of 1mA.

In some embodiments, the periodicity of such sensory percepts may correspond to a characteristic of a movement related to the movement cue provided by the neuronal stimulation signal, such as a waking pace, a breathing rhythm, a dancing rhythm etc.

In this manner, the neuronal stimulation signal may be used to provide guidance to a patient desiring to perform a periodic or rhythmic movement or behavior such as walking, breathing and / or dancing.

Further, in some embodiments, the means for transmitting the neuronal stimulation signal may be further configured to control a frequency, a pulse width, a pulse shape and / or an amplitude of the neuronal stimulation signal transmitted to the electric contact of the neuronal stimulation electrode.

In this manner, a great variety of neuronal stimulation signals can be transmitted and be used to provide a great variety of different movement cues to the individual e.g. via elicited sensory percepts in the sensory cortex. Moreover, by controlling signal parameters such as the frequency, the pulse width, the pulse shape and / or the amplitude, the neuronal stimulation signal and thus also the provided movement cue can be tailored to the individual, e.g. via carrying out calibration and learning procedures specific to the individual.

For instance, the means for transmitting may be further configured to control a movement speed, a pace regularity and/or a balance of the individual via the frequency the pulse width, the pulse shape and / or the amplitude of the neuronal stimulation signal.

In this manner the provided system enables the design of closed-loop movement enhancement systems, wherein one or more characteristics of a movement of the individual are determined and then used to provide a feedback signal for the neuronal stimulation signal. In this way, the sensory quality of the provided movement cue can dynamically be adjusted to varying external conditions and / or changing movement characteristics.

Further, the means obtaining the neuronal stimulation signal may comprise means for selecting at least one neuronal stimulation signals to be transmitted to the neuronal stimulation electrode. For instance, the means for selecting may be adapted to select at least two different neuronal stimulation signals having different frequencies.

In this manner the individual, a therapy supervisor and / or an autonomous control logic may select different stimulation signals in response to different requirements. For instance, the individual may select a different signal frequency depending on whether he wants to carry out the movement at a slow or a fast pace. In addition, the intensity of the movement cue may be adjusted to be always clearly perceivable.

In some embodiments, a first neuronal stimulation signal may be adapted to control the movement speed, the pace regularity and/or the balance of the individual and a second neuronal stimulation signal is adapted to counteract a temporary movement impairment of the individual.

For instance, the second neuronal stimulation signal may be adapted to provide a FOG breakout signal to the individual and the first a gait pacemaker signal. In this manner the same stimulation system using the same neural interface (e.g. a DBS electrode) can be used to end a FOG period and to provide a gait pacemaker signal enhancing gait quality and reducing the occurrence frequency of FOG events.

Further, the means for transmitting the neuronal stimulation signal may be adapted to transmit at least two different neuronal stimulation signals to two different contacts of the neuronal stimulation electrode, preferably simultaneously.

In this way, auxiliary information can be provided to the individual together with the movement cue. For instance, while the movement cue is applied via the first electric contact, the second contact may be used to communicate a balance signal, information about the body posture, the position of the individual with respect to a reference position or a warning signal.

Another embodiment, may further comprise means for obtaining information about the body posture of the individual, means for determining, based on the obtained information, a neuronal stimulation signal to be applied to at least one afferent axon targeting at least one sensory neuron in the cortex of the individual, wherein the determined neuronal stimulation signal corresponds to the proprioceptive information to be communicated and means for transmitting the determined neuronal stimulation signal to a neuronal stimulation means of the individual adapted to apply the determined neuronal stimulation signal to the at least one afferent axon.

Such a system can be used to provided proprioceptive information directly to the cortex of an individual either in order to substitute proprioceptive sensations that were impaired by a neurological disease or a lesion of the nervous systems of the individual or to provide artificial proprioceptive information which has no physiological counterpart. For instance, the determined neuronal stimulation signal may be configured to elicit a conscious or subconscious sensory percept in the cortex of the individual.

In contrast to the prior art the present invention enables supplementation of natural afferent proprioception by artificial means to aid the individual in the integration of movement, posture and proprioception.

For example, the information about the body posture may comprise at least one of: information about an articulation state of a joint of the individual; information about a flexing angle of a joint of the individual; information about the balance of the body of the individual; information about a tone of a muscle of the body of the individual; information about a position of a part of the body of the individual with respect to a reference position, information about a surface contact of a part of the body of the individual.

Further, the means for obtaining the information about the body posture of the individual may comprise at least one of: a pressure sensor; a tension sensor; a balance sensor; an acceleration sensor; a temperature sensor; an image sensor; a force sensor; a distance sensor; an angle sensor; a speed sensor;

By using one or more of such sensors an accurate model of the body posture of the individual may be determined and be used to determine a tailored neuronal stimulation signal that is adapted to communicate precise and reliable proprioceptive information directly to the cortex of the individual.

Further, the means for determining the neuronal stimulation signal may comprise means for accessing a data storing means storing relations, specific for the individual, between a plurality of proprioceptive information and a plurality of corresponding neuronal stimulation signals.

This embodiment greatly improves the efficiency and flexibility of communicating the desired proprioceptive information to the cortex of the individual. For instance, a communication device that interfaces with or uses the provided system can easily determine and directly transmit the specific neuronal stimulation signal corresponding to a desired proprioceptive information via stimulation of afferent axons targeting sensory neurons in the cortex of the individual.

For instance, in some embodiment of the present invention, the stored relations between the proprioceptive information and the corresponding neuronal stimulation signals may be based at least in part on one or more of: spatial information for the at least one afferent axon, spatial information for the at least one neuronal stimulation means, neuronal connectivity information for the at least one afferent axon, an electric field distribution associated with the neuronal stimulation means, functional neuroimaging data for the individual, diffusion tensor imaging data for the individual, neuroanatomical reference data being relevant for the individual, cortical excitation data for the individual, sensory perception data for the individual, behavioral data based at least in part on subjective experiences of the individual and / or an optimization procedure for maximizing the number of proprioceptive information that can be perceived by the individual.

Further, the stored specific relations may be based at least in part on proprioceptive learning data for the individual, the learning data associating the plurality of proprioceptive information with the plurality of corresponding neuronal stimulation signals.

Further, the means for determining the neuronal stimulation signal may comprise means for determining an excitation probability of the at least one afferent axon and / or the at least one sensory neuron based at least in part on the obtained spatial information, preferably by using a finite element method and / or a neuronal compartment model.

In this manner active electric properties (e.g. the non-linear neuronal excitability) of the at least one axon can be taken into account by the system when determining the neuronal stimulation signal, thereby further enhancing the specificity and accuracy of the proprioceptive information to be communicated.

In another embodiment, not covered by the appended claims, the present disclosure describes a system for communicating movement information to an individual, comprising means for providing a first neuronal stimulation signal to the cortex of an individual adapted to provide a movement cue for the individual and means for providing a second neuronal stimulation signal to the cortex of the individual adapted to provide proprioceptive information to the individual, wherein the first and the second neuronal stimulation signal are provided together to the cortex of the individual.

In many cases, neurological movement impairments also entail a degradation of physiological proprioceptive information reaching the cortex of the affected individual via the sensory nervous system. Thus, by communicating proprioceptive information together with a movement cue via a neuronal communication interface the system provided by the present invention can substantially enhance the success of movement impairment therapies.

Similar as for other embodiments discussed above, the first and / or the second neuronal stimulation signal may be applied via at least one portion of a neural stimulation electrode already implanted for a purpose different from communicating the movement information (e.g. for the purpose of applying a neuromodulation therapy to the thalamus or the sub-thalamic nucleus).

Alternatively, the first and / or the second neuronal stimulation signal may also be applied via different neural interface means such as transcranial stimulation means, sub-dural electrode arrays, spinal cord stimulation means, optogenic neuronal interface means and / or peripheral nerve stimulation means.

Further, the proprioceptive information conveyed by the second stimulation signal may be related to a body part of the individual that is involved in a movement of the individual associated with the movement information to be communicated.

For instance, the second neuronal stimulation signal comprises one of the following information: information about an articulation state of a joint of the individual; information about a flexing angle of a joint of the individual; information about the balance of the body of the individual; information about a tone of a muscle of the body of the individual; information about a position of a part of the body of the individual with respect to a reference position, information about a surface contact of a part of the body of the individual.

In this manner, the individual can be provided with relevant proprioceptive information that can assist in performing a movement task such as walking, dancing, etc. Similar to other embodiments discussed above, the first neuronal stimulation signal may comprise a signal (e.g. a pulse train signal) that is designed to be perceived as periodic by the individual.

Further, the means for providing the first and / or the second neuronal stimulation signal may be adapted to control a frequency, a pulse width, a pulse shape and / or an amplitude of the first and / or the second neuronal stimulation signal and / or adapted to control a relative timing between the first and the second neuronal stimulation signal. In this manner, the provided system can ensure that both neuronal stimulation signals can be applied to the cortex of the individual in a manner that is specific to the individual.

In particular, the second neuronal stimulation signal may be provided at a rate that is more than 2 times, preferably more than 5 times, more preferably more than 10 times and even more preferably more than 20 times larger than a frequency of the first neuronal stimulation signal.

Providing the second neuronal stimulation signal more often than the first one allows the individual to use the provided proprioceptive information while performing the movement associated with the movement cue provided by the by the first neuronal stimulation signal.

Further, the second neuronal stimulation signal may be provided quasi-continuously with respect to the first neuronal stimulation signal, e.g. at a rate that is much larger than a frequency of the first neuronal stimulation signal.

Similar as for other embodiments discussed above, the provided system may further comprise means for obtaining information about the body posture of the individual via at least one of: a pressure sensor; a tension sensor; a balance sensor; an acceleration sensor; a temperature sensor; an image sensor; a force sensor; a distance sensor; an angle sensor; a speed sensor.

Using one or more of such sensors allows the system to obtain precise information on the body posture of the individual. In several embodiments, the means for obtaining information about the body posture of the individual may be integrated into at least one of the following: a piece of apparel; a piece of footwear; a prothesis; an orthosis; an exoskeleton; an autonomous robotic companion; a wearable electronic device; an implanted device.

Further, the various systems provided by the present invention may also comprise means for receiving a neuronal measurement signal corresponding to a neuronal excitation pattern recorded via a neuronal excitation measurement device.

Further, the neuronal measurement signal may be received from at least one of the following: an electroencephalography, EEG, device; a neuro-electrode; a deep brain stimulation electrode; a sub-dural electrode; a sub-dural electrode array; a connected wearable device; a transcranial excitation measurement device.

By integrating measurements of neuronal excitation patterns, the systems provided by the present invention can be used for closed-loop and / or neurofeedback applications. For instance, the system may be configured to receive a signal corresponding to a neuronal excitation pattern associated with a movement intention and / or to motor-sensory information of the individual.

In this manner, the system could be directly controlled by the mind of the individual. For instance, instead of adjusting the walking pace via a control device such as a smartphone the individual could control the pace by merely *thinking of* walking slowly or fast. The system would then record the corresponding neuronal excitation pattern and derive the required stimulation signal parameters (e.g. frequency, pulse width amplitude etc.) that correspond to the walking pace intended by the individual. With training the individual can even regain full dynamic control over his walking pace or other movements supported by the systems provided by the present invention.

Another embodiment may further comprise: means for determining the operational state of the apparatus, means for transmitting a first neuronal stimulation signal to a neuronal stimulation means of the individual adapted to elicit a sensory percept in the cortex of the individual, wherein the first neuronal stimulation signal is indicative of the operational state of the apparatus. In this way, a neuronal stimulation apparatus can be enabled to communicate various operational status information directly to the consciousness of a person using the apparatus without relying on additional interface equipment such as a remote control or a telemetry device. Consequently, the user or patient can directly be informed when the performance of the apparatus deteriorates or is about to deteriorate.

For instance, the operational state of the apparatus that is indicated to the individual may comprises one or more of the following: a fault state of the apparatus; a charging state of a power source of the apparatus; a normal operation state of the apparatus; a maintenance state of the apparatus; a connectivity state of the apparatus; a communication state of the apparatus; a power transfer state between the apparatus and an external charging device.

For instance, in some embodiments, the system may be configured to inform the user / patient of a low battery state of the apparatus, before the battery charge level drops below a threshold value required for proper functioning of the apparatus. In this way the user can duly schedule a replacement or recharging of the battery, without risking his symptoms to become untreated. For instance, the system could be configured such that the device sends out a sequence of patient-perceptible somatosensory experiences (paresthesias) to alert the patient of the low-battery state (e.g. by inducing a feeling of vibration alternating between the left and the right hand of the patient) essentially providing a patient-internal alert which manifests as physical sensations.

Further, if the neuronal stimulation apparatus is a neuronal communication apparatus or a brain-computer interface device, the user can for instance be informed when a wireless signal quality deteriorates or increases and /or when a communication interface of the apparatus has received a message or information to be presented to the user.

Similarly, if the neuronal stimulation apparatus and / or its power source (such as a battery) may be implanted subcutaneously, the operational state may comprise a power transfer state between the apparatus and an external charging device. Such an external charging device may be configured to charge the power source of the apparatus wirelessly e.g. via electromagnetic induction. For instance, the means for determining the operational state of the apparatus may determine a relative position between the external charging device and the subcutaneously implanted power source of the neuronal stimulation apparatus. The means for transmitting the first neuronal stimulation signal may then transmit and / or generate a neuronal stimulation signal that indicates a relative position between the charging device and the apparatus that is suitable for wireless charging of the power source. Such a neuronal stimulation signal may either be binary, i.e. it may indicate whether the relative position is suitable for charging or not. Alternatively, it may also be (quasi-) continuous allowing the individual to gradually optimize the charging position in order to maximize power transfer between the external charging device and the power source of the apparatus. For instance, in some embodiments the system may further comprising means for transmitting a second neuronal stimulation signal indicative of an imminent transmission of the first neuronal stimulation signal intended for perception and/or interpretation by the individual and / or means for transmitting a third neuronal stimulation signal indicative of a completed transmission of the first neuronal stimulation signal.

In this way, a neuronal stimulation apparatus can first inform the user / patient that status information is to be transmitted. The user can then focus his attention to the imminent sensory percept to be elicited by the first neuronal stimulation signal. After the information carried by the first neuronal stimulation signal is perceived, the third neuronal stimulation signal may then inform the user / patient that no special attention focus is required anymore.

Further, in some embodiments, the means for determining the operational state of the apparatus may comprise one or more of the following: voltage, current and / or power measurement circuitry; a temperature sensor; a magnetic field sensor; signal analysis circuitry; a timer; connectivity measurement circuitry; circuit analysis circuitry; wireless packet data analysis circuitry; neuronal excitation measurement circuitry; power transfer measurement circuitry.

In this manner, the system may be enabled to determine various operational states of the apparatus and provide the user / patient with precise and detailed status information.

Further, the system described above may also comprise at least one of: means for generating the first and / or the second neuronal stimulation signal; an auxiliary power source; auxiliary digital signal processing circuitry; auxiliary memory circuitry storing at least one neuronal stimulation signal or signal parameters for generating at least one neuronal stimulation signal; signal multiplexing and / or switching circuitry; an auxiliary signal amplifier for amplifying the first and / or the second neuronal stimulation signal; magnetic and / or thermal shielding equipment; means for obtaining the first and / or the second neuronal stimulation signal from memory circuitry or via a communication interface of the apparatus.

In this way, the system may provide redundant hard- and software components that provide the required functionalities for signaling the device status information via the neuronal stimulation signal to the individual. For instance, even if the primary battery of the neuronal stimulation apparatus is completely empty, the auxiliary power source could provide enough energy for generating and transmitting the required neuronal stimulation signal indicating to the individual that the primary battery is empty.

Further, the means for generating the first neuronal stimulation signal may also comprises means for accessing a data storing means storing relations, specific for the individual, between a plurality of operational states and a plurality of corresponding neuronal stimulation signals.

In this manner, different device statuses can be encoded by different, patient specific neuronal stimulation signals that may be tailored to the patient's neurophysiology and / or determined via a perceptual learning procedure associating different sensory percepts with different operational states of the apparatus.

Further, the first and / or the second neuronal stimulation signal may be characterized by a plurality of signal parameters such as a pulse frequency, a pulse polarity, a pulse shape, a pulse amplitude and / or a pulse width. In particular, different combinations of signal parameters may correspond to different operational states of the apparatus to be indicated to the individual.

Further, in some embodiments, the neuronal stimulation means may comprise one of the following: a brain stimulation electrode in particular a deep brain stimulation electrode; a subdural stimulation electrode or subdural electrode array; a subdural microwire or microwire array; a transcranial stimulation device; an unused contact of a neuromodulation electrode; an artificial synapse; an opto-neuronal interface device; a chemical-neuronal interface device; a neural surface mesh; an injectable neural interface; and an artificial brain part, such as e.g. an artificial hippocampus.

In some embodiments, the first and /or the second neuronal stimulation signal may be adapted to elicit action potentials in at least one afferent axon targeting the sensory cortex of the individual. In particular, the first, the second and / or the third neuronal stimulation signal may be adapted to elicit a sensory percept in a portion of the cortex of the individual associated with a specific sensory modality.

Specifically, said portion of the cortex may be one or more of the following: a somatosensory cortex area; an auditory cortex area; a visual cortex area; an olfactory cortex area; and an entorhinal cortex area or components of the circuit of Papez.

By targeting afferent axons directly targeting the cortex of the individual a precise and fine-grained sensory specificity can be achieved for the different neuronal stimulation signals. For instance, different battery levels may be mapped to different areas within palm of the right hand of the patient by inducing action potentials in different subpopulations of axons targeting the sensory cortex of the right hand. In another embodiment the present invention provides a neuronal stimulation apparatus comprising any of the systems described above.

For instance, the systems described above may be used with neuronal stimulation apparatuses such as: a deep brain stimulation apparatus; a neuromodulation apparatus; a neuronal communication apparatus; an electrical pacemaker apparatus; a transcranial stimulation apparatus; a brain-machine-interface apparatus; a peripheral nervous system stimulator; a vagus nerve stimulator; a spinal cord stimulator; a receptor (e.g. baroreceptor) or synapse stimulator or modulator; a responsive neurostimulation device with integrated sensors; a neuroprosthetic device;

In a further embodiment, not covered by the appended claims, the present invention provides a method for communicating an operational state of a neuronal stimulation apparatus to an individual, comprising: determining the operational state of the apparatus, transmitting a neuronal stimulation signal to a neuronal stimulation means of the individual adapted to elicit a sensory percept in the cortex of the individual, wherein the first neuronal stimulation signal is indicative of the determined operational state of the apparatus.

Such a method may further comprise a step of transmitting a second neuronal stimulation signal indicative of an imminent transmission of a first neuronal stimulation signal and / or a or a step of transmitting a third neuronal stimulation signal indicative of a completed transmission of the first neuronal stimulation signal.

As already discussed above, the operational state of the apparatus may comprise one or more of the following: a fault state of the apparatus; a charging state of a power source of the apparatus; a normal operation state of the apparatus; a maintenance state of the apparatus; a connectivity state of the apparatus; a communication state of the apparatus.

In addition, such a method may further comprise a step of: generating the first, the second and / or the third neuronal stimulation signal based on accessing a data storing means storing relations, specific for the individual, between a plurality of operational states and a plurality of corresponding neuronal stimulation signals.

Specifically, the specific relations may be based at least in part on one of the following: conceptual learning data for the individual; neuro-imaging data for the individual; electrophysiological measurement data for the individual; neuronal connectivity information for the individual; electric field simulation data for the neuronal stimulation means of the individual; neuronal excitability model data for the individual.

In this manner, even complex device status information such as a wireless connection signal quality indicator can be associated with corresponding sensory percepts that are specific for each individual.

The present disclosure also describes a computer program, comprising instructions to perform any of the methods described herein, when executed by the signal processing circuitry of a neuronal stimulation apparatus such a one of the neuronal stimulation apparatuses mentioned above.

The present disclosure also relates to an electronic information processing device, comprising a system according to any of the embodiments discussed above. For instance, the functionalities of any of the above systems may be implemented on a general-purpose signal processing device such as a smartphone comprising memory, digital and analog signal processing circuitry as well as interface means (e.g. a wireless communication interface) that allows to transmit neuronal stimulation signals to various kinds of neural interface means such as a DBS device.

The present disclosure also describes a distributed electronic information processing system, comprising the system according to any of the embodiments discussed above. For instance, some system components may not be collocated in a single device but may be communicatively connected via a wired or wireless communication interface.

In another embodiment, the present invention provides a computer program, comprising instructions to implement the functionalities of a system according to any of the embodiments discussed above, when being executed by an electronic information processing device or a distributed electronic information processing system.

### 4. Short Description of the Figures

Aspects of the present invention are described in more detail in the following by reference to the accompanying figures. These figures show:
- **Fig. 1**: a diagram illustrating a neuronal stimulation electrode for stimulating afferent axons targeting the sensory cortex of an individual. The neuronal stimulation electrode can be interfaced with a neuronal stimulation system according to an embodiment of the present invention;
- **Fig. 2**: a block diagram of a neuronal stimulation signal generator for driving a neuronal stimulation electrode which can be interfaced with a neuronal stimulation system according to an embodiment of the present invention neuronal stimulation signals;
- **Fig. 3**: a block diagram of a neuronal stimulation system according to an embodiment of the present invention;
- **Fig. 4a**: a diagram illustrating the operation of a neuronal communication system according to an embodiment of the present invention;
- **Fig. 4b**: a workflow diagram for a procedure for establishing a neuronal communication interface with a human individual;
- **Fig. 5**: a diagram illustrating the encoding of conceptual information using a neuronal communication system according to an embodiment of the present invention;
- **Fig. 6**: a diagram illustrating the encoding of conceptual information using a neuronal communication system according to an embodiment of the present invention;
- **Fig. 7**: a diagram illustrating the encoding of conceptual information using a neuronal communication system according to an embodiment of the present invention;
- **Fig. 8**: a diagram illustrating a parameter space for a neuronal stimulation signal provided by a neuronal stimulation system according to an embodiment of the present invention;
- **Fig. 9**: a diagram illustrating a choice of signal parameters for a neuronal stimulation signal provided by a neuronal stimulation system according to an embodiment of the present invention;
- **Fig. 10**: a diagram illustrating a conceptual learning procedure for learning relations between sensory percepts and conceptual information to be communicated by a neuronal stimulation system according to an embodiment of the present invention;
- **Fig. 11**: a block diagram of a calibration and learning procedure for establishing relations between neuronal stimulation signal and corresponding conceptual information to be communicated by a neuronal stimulation system according to an embodiment of the present invention;
- **Fig. 12**: a diagram illustrating the operation of a neuronal stimulation system according to an embodiment if the present invention that can be interfaced with at two neuronal stimulation electrodes targeting afferent axons of the human brain;
- **Fig. 13**: an illustration of a therapeutic multi-contact neuromodulation electrode adapted for modulation of efferent motor axons. Unused contacts of the electrode can also be used for stimulating afferent axons targeting the sensory cortex of an individual via a neuronal stimulation system according to an embodiment of the present invention;
- **Fig. 14**: a diagram illustrating an individual operating a neuronal stimulation system according to an embodiment of the present invention;
- **Fig. 15**: a diagram illustrating a neuronal stimulation electrode for stimulating afferent axons targeting the sensory cortex of an individual. The neuronal stimulation electrode can be interfaced with a neuronal stimulation system according to an embodiment of the present invention;
- **Fig. 16**: a diagram illustrating a therapeutic multi-contact neuromodulation electrode adapted for modulation of brain nuclei associated with a movement impairment. Unused contacts of the electrode can be used for stimulating afferent axons targeting the sensory cortex of an individual via a neuronal stimulation system according to an embodiment of the present invention;
- **Fig. 17A**: a block diagram of a neuronal stimulation signal generator for driving a neuronal stimulation electrode which can be interfaced with a neuronal stimulation system according to an embodiment of the present invention;
- **Fig. 17B**: a block diagram of a neuronal stimulation system according to an embodiment of the present invention;
- **Fig. 18**: a diagram illustrating how a movement cueing channel can be implemented using a neuronal stimulation system according to an embodiment of the present invention;
- **Fig. 19**: a diagram illustrating exemplary neuronal stimulation signals adapted to provide a movement cue and a FOG break-out signal;
- **Fig. 20**: a diagram illustrating an individual operating a neuronal stimulation system according to a further embodiment of the present invention;
- **Fig. 21**: a diagram illustrating how a proprioceptive information channel can be implemented using a neuronal stimulation system according to an embodiment of the present invention;
- **Fig. 22**: a diagram illustrating how the proprioceptive information channel of Fig. 21 can be used to communicate information on the articulation state of a knee joint of an individual;
- **Fig. 23**: a diagram illustrating the relative timing of the movement cue channel of Fig. 19 and the joint articulation state channel of Figs. 21 and 22 while the individual depicted in Fig. 20 performs a walking task;
- **Fig. 24**: a block diagram of a neuronal stimulation apparatus / device comprising a system according to an embodiment of the present invention;
- **Fig. 25**: a block diagram of an operational state circuit that may be part of or be used with a system according to an embodiment of the present invention;
- **Fig. 26**: a block diagram of a neuronal stimulation apparatus / device comprising a system according to an embodiment of the present invention;
- **Fig. 27**: a diagram illustrating how a binary battery charge status channel can be implemented using a neuronal stimulation system according to an embodiment of the present invention;
- **Fig. 28**: a block diagram of a neuronal stimulation apparatus comprising a system according to an embodiment of the present invention adapted to monitor and signal operational status information;
- **Fig. 29**: a block diagram of an operational state circuit that may be part of or be used with a system according to an embodiment of the present invention;
- **Fig. 30**: a diagram illustrating how a communication status signal and a device fault signal can be implemented using a neuronal stimulation system according to an embodiment of the present invention;
- **Fig. 31**: a block diagram of a neuronal stimulation device / apparatus comprising a system according to an embodiment of the present invention configured for monitoring whether the output signals of neuronal stimulation device are within specified parameter ranges;
- **Fig. 32**: a diagram illustrating how a (quasi-) continuous communication channel for signaling a battery charging level can be implemented using a neuronal stimulation system according to an embodiment of the present invention;
- **Fig. 33**: a diagram illustrating how a (quasi-) continuous communication channel for signaling a wireless connectivity status can be implemented using a neuronal stimulation system according to an embodiment of the present invention;

### 5. Detailed description of some exemplary embodiments

In the following, exemplary embodiments of the present invention are described in more detail, with reference to neuronal stimulation and / or communication systems that can be interfaced with neuronal stimulation electrodes such as deep brain stimulation (DBS) electrodes. However, the systems provided by the present invention can also be used with different neuronal stimulation means (e.g. opto-neuronal) that are capable to stimulate afferent axons targeting the sensory cortex of an individual. While specific feature combinations are described in the following with respect to the exemplary embodiments of the present invention, it is to be understood that the disclosure is not limited to such embodiments. In other words, not all features have to be present for realizing the invention, and the embodiments may be modified by combining certain features of one embodiment with one or more features of another embodiment. Specifically, the skilled person will understand that features, components and / or functional elements of one embodiment can be combined with technically compatible features, components and / or functional elements of any other embodiment of the present invention.

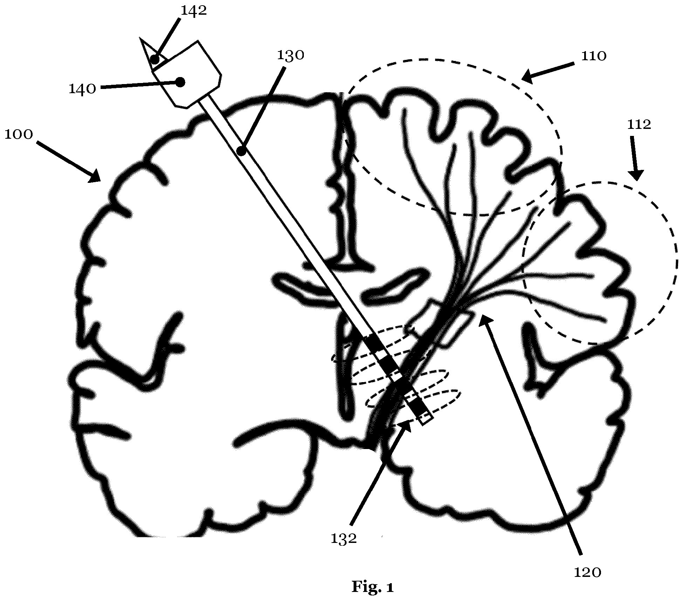

Figure 1 depicts a diagram illustrating a neuronal stimulation electrode 130 for stimulating afferent axons 120 targeting sensory neurons in the cortex 100 of a human brain. The afferent axons 120 target different areas 110, 120 of the cortex 100 that may be related to different sensory modalities (e.g. touch, temperature sense, vision, hearing, etc.) and / or different body regions (e.g. cochlea, retina, hand, tongue, foot etc.) from which the respective sensory modality is perceived by the respective area of the cortex. For instance, the cortical area 110 may be a somatosensory area of the tongue and the cortical area 112 may be a somatosensory area of the left hand.

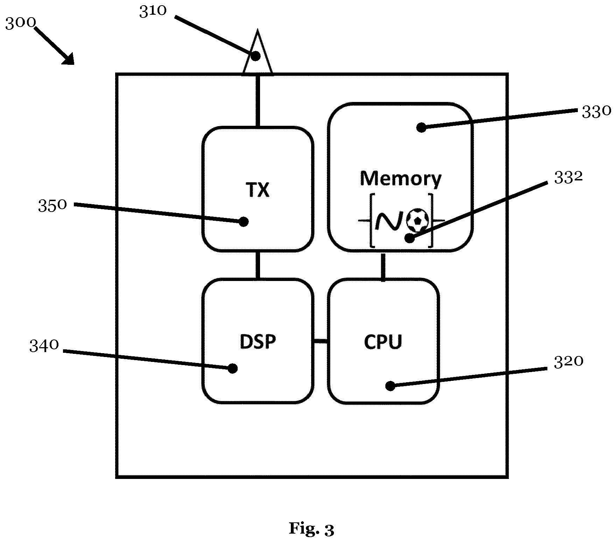

The afferent axons 120 are connected via synapses (not shown) with their respective target neurons in the respective sensory area 110, 112 of the cortex 100. For instance, the axons 120 may be thalamocortical axons relaying sensory information from the thalamus to the cerebral cortex 100. The neuronal stimulation electrode 130 comprises a plurality of independently controllable electric contacts 132 that are arranged in the vicinity of a bundle of afferent axons targeting the sensory areas 110 and 112 of the cerebral cortex 100. In the illustrated example, the neuronal stimulation electrode 130 is connected to a neuronal stimulation signal generator 140, which is adapted to apply neuronal stimulation signals to the afferent axons 120 via the independently controllable electric contacts 132 of the neuronal stimulation electrode 130. In addition, the neuronal stimulation electrode 130 may further comprise a wireless interface 142 for interfacing the signal generator 140 with a neuronal stimulation system (not shown, see Fig. 3) which may be adapted to determine the waveform and / or the signal parameters (e.g. pulse width, pulse shape, frequency, amplitude, number of pulses etc.) of the neuronal stimulation signals that are generated and applied by the signal generator 140 to the afferent axons 120 via the stimulation electrode 130.

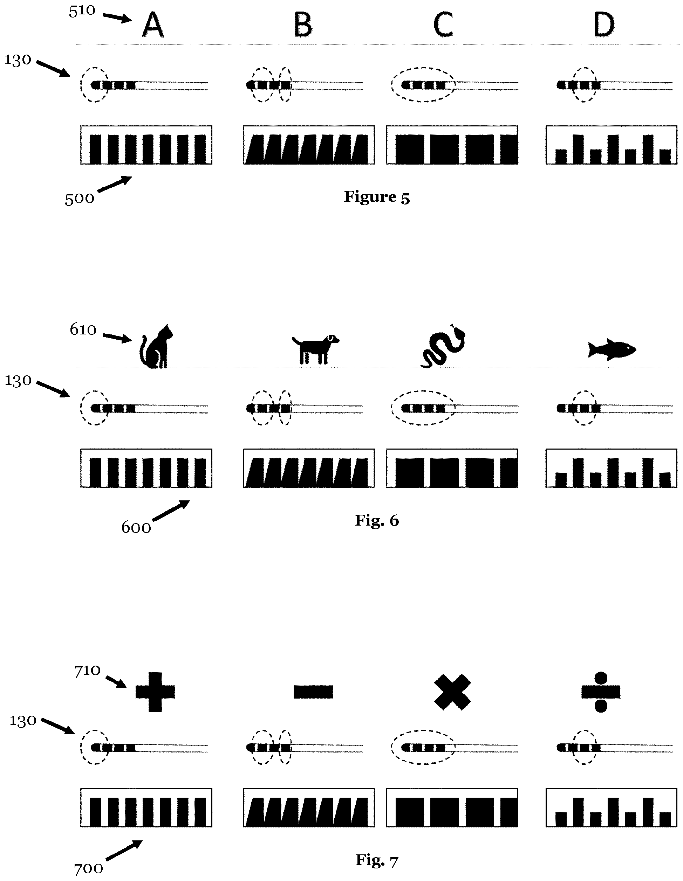

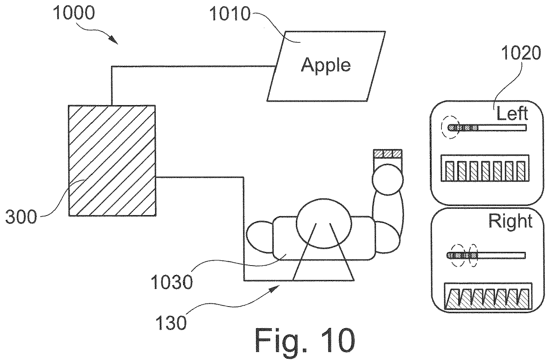

For instance, the neuronal stimulation system may determine the waveform and / or the signal parameters of the neuronal stimulation signal such that a desired sensory percept is elicited in a desired area of the sensory cortex of the individual. In some embodiments of the present invention, the cortex 100 of the individual which is receiving the neuronal stimulation signal (i.e. via afferent action potentials of the stimulated afferent axons 120) may associate the corresponding sensory percept with conceptual information such as a letter, a word, an object, a direction, etc. For example, similar to learning how to understand Morse code, the individual may have previously participated in a conceptual learning procedure (e.g. see Fig. 10) establishing an associative link between a given sensory percept elicited by a given stimulation signal and a corresponding piece of conceptual information (e.g. see Figs. 5 - 7) that is to be communicated to the individual via the neuronal stimulation electrode 130.

In this approach no nuclei or neuron-rich grey matter are preferably targeted by the neuronal stimulation electrode 130 but preferably the axon-rich white matter of the brain, which contains the information transmitting pathways the brain uses for natural neural communication. In this manner, the present invention provides a white-matter computer-brain-interface (CBI), i.e. a system that generates and provides electrical signals the brain can interpret as meaningful input. Maximal efficiency for such a white-matter CBI would be accomplished through perfectly selective recruitment of single axonal fibers 120.

In other embodiments of the present invention the electrode 130, the signal generator 140 and / or the wireless interface 142 may also be part of an integrated neuronal stimulation and /or communication system, e.g. if said components are customized for neuronal communication purposes, i.e. for direct neuronal communication. For instance, a neuronal communication system may comprise of specialized communication software running on a multi-purpose computation device such as a smartphone and a customized assembly of signal generator 140 and stimulation electrode 130 which communicate with the multi-purpose communication device via the wireless interface 142 using conventional wireless data transmission technology such as Wi-Fi, Bluetooth and /or NFC.

In other embodiments of the present invention the neuronal stimulation electrode 130 may be directly connected via conducting wires to a neuronal stimulation system comprising a data processing system and a signal generator similar to the signal generator 140. In this case the wireless interface 140 is not needed.

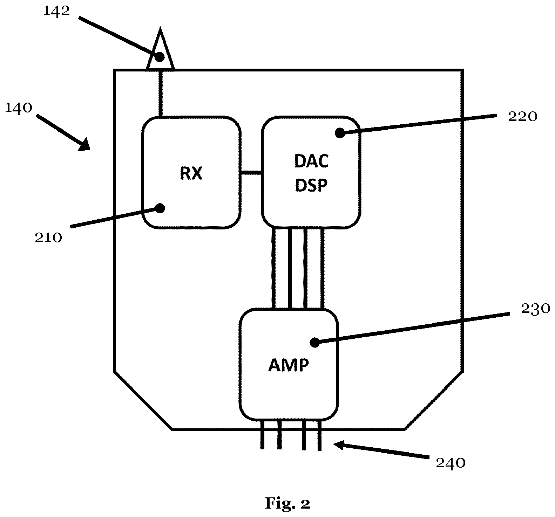

Figure 2 is a block diagram of a neuronal stimulation signal generator 140 which can be used to apply neuronal stimulation signals to afferent axons 120 via a neuronal stimulation electrode such as the stimulation electrode 130 of Fig. 1**.** The neuronal stimulation signal generator 140 may comprise a wireless interface 142 for communicating with a remote neuronal stimulation system (e.g. see Fig. 3) which may be adapted to determine, to select and to transmit a waveform and / or signal parameter of the neuronal stimulation signal to the signal generator 140 in order to elicit a desired sensory percept as described above with reference to Fig. **1****.**

For instance, the neuronal stimulation signal generator 140 may receive digital data packets specifying a desired neuronal stimulation signal via the wireless interface 142. Receiver (RX) circuitry 210 may process (e.g. filter, amplify, mix, down-convert to baseband etc.) the received digital data packets and feed the processed digital data packets to a digital signal processor (DSP) 220 with may comprise an integrated digital-to-analog converter (DAC). The DSP then processes the digital data packets to generate one or more neuronal stimulation signals which may then be amplified and applied to a neuronal stimulation electrode such as electrode 130 of Fig. 1 by an output amplifier (AMP) 230. For instance, the output AMP 230 may for instance be configured to drive four (or any other number) independently controllable electric contacts 132 of a stimulation electrode such as electrode 130 of Fig. 1 via four wires 240.

In other embodiments, the DSP 220 may receive the digital data packets specifying the neuronal stimulation signal also via a wire-based interface or directly from a collocated digital data packets processing circuit (e.g. a CPU) which is adapted to determine the waveform and / or signal parameters of a desired neuronal stimulation signal corresponding to a desired sensory percept to be elicited and / or a desired piece of conceptual information to be communicated to the individual via the neuronal stimulation electrode 130.

Figure 3 depicts a block-type circuit diagram of a neuronal stimulation and / or communication system 300 according to an embodiment of the present invention. The neuronal stimulation and / or communication system 300 may for instance comprise a wireless interface 310 and transmitter (TX) circuitry 350 for communicating (e.g. via Bluetooth technology) with a neuronal signal generator such as the generator circuit 140 described above with reference to Fig. 2. The TX circuitry 350 may be adapted to process (i.e. filter, modulate, mix, amplify, and / or upconvert) digital data packets to be communicated via the wireless interface 310. The neuronal stimulation and / or communication system 300 may further comprise a digital signal processor (DSP) 340 operably connected with the TX circuitry 350 and adapted to provide digital data packets specifying the waveform and / or the signal parameters (e.g. frequency, phase, pulse width, pulse amplitude, pulse shape, channel count, etc.) of a desired neuronal stimulation signal to be applied via a neuronal stimulation electrode such as the electrode 130 of Fig. 1 and / or a neuronal stimulation signal generator such as signal generator 140 of Fig. 1 and Fig. 2.

The neuronal stimulation and / or communication system 300 may further comprise a general data processing circuitry such as a CPU 320 operably connected to the DSP 340 and at least one digital memory device 330 operably connected to the CPU 320.

The CPU 320 and the memory 330 may interact to determine a desired neuronal stimulation signal corresponding to a desired sensory percept and / or a corresponding piece of conceptual information associated with a sensory percept.

For instance, the memory 330 may contain a personalized communication library for the individual, the library storing relations 332 between a plurality of conceptual information blocks and a plurality of corresponding neuronal stimulation signals. For instance, the memory 330 may store such a relation 332 for every letter of the alphabet and / or each number between zero and ten. In other embodiments of the present invention, the memory 330 may also store relations between stimulation signals and other types of conceptual information such as objects, colors, directions, etc.

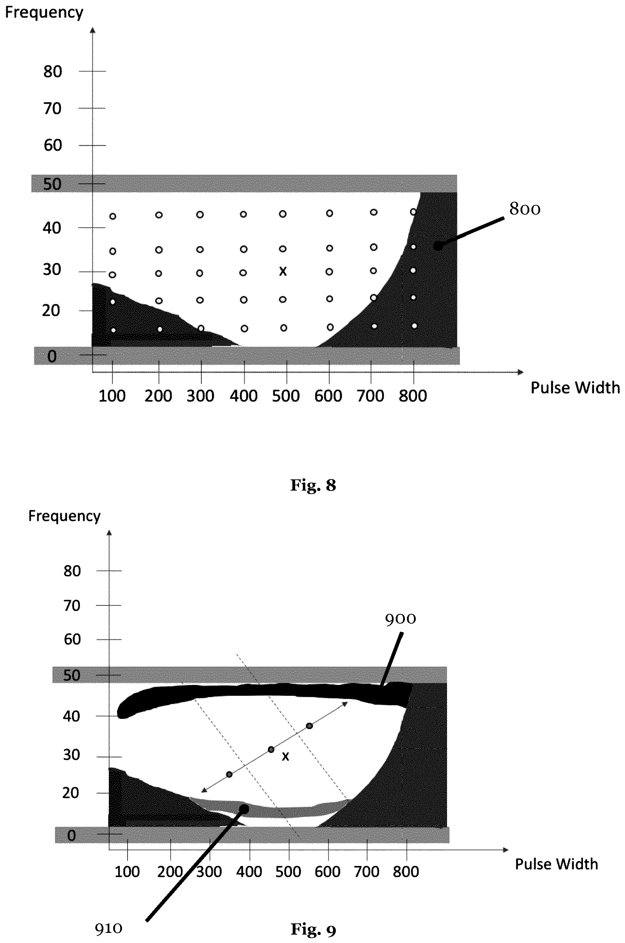

A key concept of the present invention is the calibration of such a stimulation library for each individual through both, neuroimaging and individualized testing of the individual. Neuroimaging may first be used to identify theoretically possible ranges of activation for an individual stimulation electrode while individualized testing determines which points in the parameter space of stimulation signal parameters (for details see Fig. 8 and 9 below) have the potential to be perceived and decoded by the cortex of the individual. It should be emphasized that conscious individualized testing of an individual is merely one specific example how to generate the individualized relations 332 stored in the memory 330. In other embodiments such relations 332 may also be obtained from unconscious patients, e.g. through the non-invasive observation of corresponding functional MRI (fMRI) responses on the somatosensory cortex or EEG recordings.

Further, once or while the communication library (i.e. the plurality of relations 332 stored in the memory 330) is established or is being established for an individual a specific training procedure (see Fig. 10) can be executed (again not necessarily in a conscious individual). As long as the cortex of the individual responds to classical conditioning pair learning can be executed. In this context, such a pair consists of a given sensory percept corresponding to a given neuronal stimulation signal and a piece of conceptual information to be associated with said given sensory percept and the corresponding neuronal stimulation signal).

Importantly, the type of information to be conveyed via the neuronal stimulation and / or communication system 300 whether it is visual, conceptual, categorical, auditory etc. can be chosen freely. Any information or message which can be broken down into message blocks (i.e. pieces of conceptual information that can be decoded by the cortex of an individual) can be transmitted. This includes continuous signal sources such as signals needed for e.g. an artificial balance, orientation signals or other measurement (e.g. altimeter) signals. Learning paradigms for continuous signals deviate from classical conditioning, since they involve more interactive training scenarios where utilization of the signal is a relevant success factor (e.g. orientation in an artificial virtual environment using the input signal). Continuous signals (e.g. intensity) also deviate from signal configurations for messages containing sequentially delivered message blocks (e.g. letters in a word associated with the sensory percepts the individual is trained to associate with the respective letter). In the case of continuous signals, intensity (i.e. the one-dimensional information to be transmitted) might be coded via either pulse width or frequency variations (or combinations of the two), while not varying the location and target areas in the sensory cortex targeted by the recruited axon fibers.

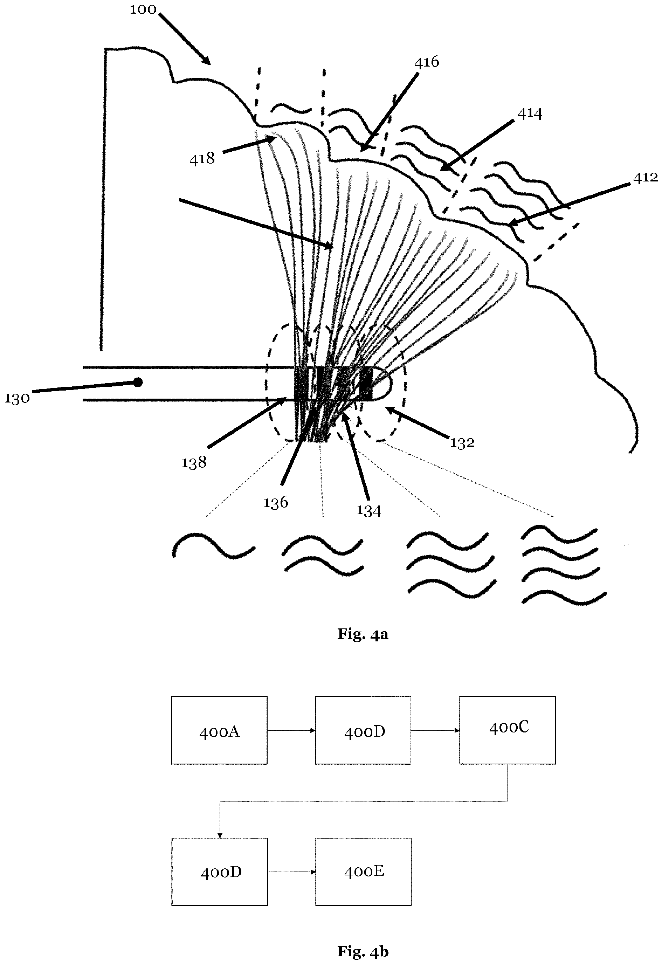

Figure 4 illustrates the operation of a neuronal communication system according to an embodiment of the present invention. For reasons of clarity, the operation is described in a simple setup wherein a single neuronal stimulation electrode 130 comprising four independently controllable electric contacts 132 - 138 is implanted such into the white-matter region of the brain of an individual that four distinct areas 412 - 418 of the sensory cortex can be stimulated via stimulating a plurality of afferent axons 120 targeting sensory neurons in the respective area 412 - 418 of the cortex 110 of the individual. For instance, the afferent axons 120 may be thalamocortical axons belonging to the visual, auditory and / or somatic sensory system of the individual. Due to the cortico-topic organization of the afferent axons 120 a one-to-one correspondence between each electric contact 132 - 138 of the stimulation electrode 130 and a corresponding area 412 - 418 of the sensory cortex 100 of the individual can be established. For instance, the waveform and / or the signal parameters of the neuronal stimulation signal that is applied to the contact 132 of the electrode 130 may be determined (e.g. by the neuronal stimulation system 300 of Fig. 3) such that action potentials are only or predominantly elicited in the specific sub-population of the afferent axons 120 that are targeting the desired area 412 of the sensory cortex.

For instance, the area 412 (414, 416, 418) may be linked to / responsible for perceiving touch percepts of the right thumb (index finger, middle finger, ring finger) of the individual. In this sense, the neuronal stimulation electrode 130 of Fig. 4a can be used to establish a four-channel CBI. In this context, the term *"channel"* is to be understood broadly such that it encompasses any a distinct communication channel to the cortex of an individual over which the cortex is capable of receiving input that can be separated from other such channels.

Because cortical organization varies from individual to individual the neuronal communication library (and the neuronal stimulation signals stored therein) described above with respect to Fig. 3 is preferably established and customized separately for each individual. For instance, from localizing the electrode 130 in post-operative imaging, identifying the contacts 132 - 138 and fusing this information with DTI-based individual tractography, a neuronal stimulation and / or communication system such as system 300 of Fig. 3 can automatically calculate spatially distinct volumes of tissue activated (VTAs) which can then be used to elicit action potentials of the axon fibers 120 recruited by the stimulation electrode 130. In the shown example, the number of selectively stimulate-able axon fiber populations equals the number of channels (i.e. four) which can be used to send information (i.e. sensory percepts and / or associated pieces of conceptual information) to the respective sensory cortex area 412 - 418 for interpretation.

In some embodiments the stimulation of the afferent axons 120 may happen in intensities which are eventually under the perception threshold of the individual. In other words, the corresponding sensory percepts are subconscious. The higher the number of distinct fiber pathways terminating in corresponding cortical areas which can be uniquely and selectively stimulated via activated axon fibers 120 around the active contacts 132 - 138 of the stimulation electrode 130, the more distinct binary signals and / or multi-symbol communication channels can be established.

A binary signal or signal channel is a volume of tissue (axon fibers 120) that is activated or not by a given neuronal stimulation signal (e.g. by a train of current pulses applied form the neuronal stimulation and / or communication system 300 via the neuronal signal generator 140 and / or the neuronal stimulation electrode 130.

Those activated axonal fibers 120 may lead to somatotopically and / or retinotopically organized areas of the sensory cortex. The recruited axonal fibers drive one specific part of the body representation on the cortex into activity, that then in turn can be decoded by the brain and associated with the desired sensory percept and / or conceptual information to be communicated. The activation can be binary (on vs. off or on vs. no stimulation at all) or continuous by modulating the signal strength via frequency and / or amplitude within a defined range (see Fig. 8 and 9 below).

The workflow illustrated in Figure 4b summarizes some of the steps that may be performed to establish a neuronal communication channel via a neuronal stimulation electrode such as electrode 130. Note that some or all the steps 400A - 400E may be executed and / or facilitated by the neuronal stimulation and / or communication systems provided by the present invention:
1**.** 400A: Tractographically reconstruct axonal fibers connecting e.g. the thalamus to the cortical area in question from DTI neuroimaging data of the subject. Said cortical area (and the fibers leading there) should have a somatotopic, retinotopic, etc. organization (this approach works for axons targeting the somatosensory cortex, auditory cortex, V1/V2 cortex etc.).
2. 400B: From post-operative CT or MRI imaging of the individual, localize the neuronal stimulation electrode 130 and model the geometric properties and positions of the electric contacts 132 - 138.
3. 400C: Calculate the maximum current pulse amplitude (e.g. measured in mA) for which a VTA reaches the outer boundary of the fiber envelope around the electrode 130 for every single electric contact 132 - 138 (e.g. considering which parts of the axons 120 can be stimulated without causing any unwanted side effects).
4. 400D: The resulting VTAs add up to establish the communication envelope of the electrode 130.
5. 400E: Optimal communication is established by stimulating maximally distinct / separate regions of the cortex in question, i.e. areas 412 - 416 via the axon fibers 120 leading to the respective area.
The minimal axon fiber count / bundle diameter is the lower boundary that defines how small a VTA can be to still recruit enough axon fibers 120 to enable communication, ideally each bundle terminates in a different partition of the sensory cortex (which parcellates in a somatotopic fashion).

It should be noted that based on surgical success every electrode location may offer a different bandwidth for communication. The possible bandwidth is determined on the basis of two functions, first the function underlying the step 400D (e.g. if no contacts are in the vicinity of the axon fibers 120 no stimulation can be applied, and no communication channel can be established) and secondly, step 400E.

The function for step 400E characterizes electrode position with respect to the possibility to uniquely stimulate distinct axon fiber populations. As such, not only the position of the stimulation electrode 130, but also the type of electrode (ring-based or directional or other) and the range of possible stimulation patterns play a role (only cathodic or also bipolar stimulations, biphasic stimulations, multi-area stimulation, MICC = multiple independent current control etc.). For instance, systems with MICC have a unique signal source connected to each individual electrode contact 132 - 138, thereby enabling the neuronal stimulation and / or communication system 300 of Fig. 3 to stimulate with fractionalized currents (e.g. put 20 % of the charge on contact 1 and 80 % on contact two at the same time) but also to perform simultaneous multi-area stimulation (e.g. stimulate with pulse amplitudes of 3 mA at 20 Hz on contact 132 and with 2 mA at 120 Hz at contact 134 of the same electrode 130).

Figures 5 - 7 illustrate how different neuronal stimulation signals 500, 600, 700 and / or VTAs can be linked by the cortex of an individual to a variety of different types of conceptual information 510, 610, 710 such as different letters, different types of animals and / or different types of abstract mathematical operators. VTA shapes are generated by defined cathode/anode and current configurations, pulse shapes can be blocks, ramps or combinations thereof and may have different frequencies, pulse lengths or alternating stimulation intensities. In the training / calibration phase (see Fig. 10 below) the sensory percepts elicited via a given neuronal stimulation signal and the corresponding VTA shape can be associated with virtually any type of conceptual information such as letters (e.g. ABC), directions (e.g. left, right, half-left, half-right) or concepts (e.g. stop, go, danger) etc.

Figures 8 illustrates a simple two-dimensional parameter space for a neuronal communication channel such as the channel provided by one of the electrode contacts 132 - 138 of the neuronal stimulation electrode 130 illustrated in Fig. 1 and Fig. 4a. In this example, the signal parameters are the pulse frequency and the pulse width for a sequence of current pulses such as the pulse sequences shown in Figs. 5 - 7.

The gray shaded area around 0Hz indicates that in this region the frequency of the stimulation signal is too low to elicit any sensory percept in the corresponding region of the sensory cortex of the individual. The gray shade area around 50Hz indicates another region that is not suited for eliciting meaningful sensory percepts.

Each of the small white circles in Fig. 8 indicate a possible combination of frequency and pulse width that may be used to elicit a distinct (i.e. distinguishable) sensory percept within the cortex area, e.g. area 412 in Fig. 4a corresponding to the respective electric contact, e.g. contact 132 in Fig. 4a, that is used to apply the neuronal stimulation signal characterized by said combination of frequency and pulse width.

The dark gray shaded area 800 indicates parameter ranges that are also not suited for neuronal communication because stimulation signals corresponding to these ranges may result in a degraded capability of the individual to clearly distinguish and / or localize the corresponding conscious sensory percept.

Figure 9 illustrates how a communication channel with three distinct symbols (e.g. low middle and high or 1, 2, 3) can be implemented by using three different combinations (indicated by the dark small circles) of signal parameters. The parameter regions 900 and 910 have also been determined to be unsuited for neuronal communication e.g. because of the corresponding stimulation signal inducing pain or being below the consciousness threshold. In this context it should be highlighted that already three such neuronal communication channels, each capable of signaling three symbols, are sufficient to communicate 3³=27 different pieces of conceptual information. In other words, a single stimulation electrode such as electrode 130 of Fig. 1 and Fig. 4a having three independently controllable electric contacts may be sufficient to directly communicate each letter of the alphabet and a stop symbol to the individual via direct neuronal communication. For this neuronal communication paradigm to work properly, similar to learning Morse code, the individual has to learn the respective associations between the sensory percepts elicited by each of the 27 different neuronal stimulation signals and each letter of the alphabet.

To stay in the above example, a communication signal can be generated by stimulating the respective electrode contact 132, 134, 136, 138 affecting axon fibers 120 leading to the respective cortex area 412, 414, 416, 418 either one-by-one (essentially training the cortex to associate a signal with a body part in a binary fashion - the part is stimulated or not) or in combination with weak, moderate or strong stimulation sent in combination.

Individually, the levels of stimulation of a specific axon fiber bundle 120 and the associated body part which the individual patient can differentiate (e.g. can the individual tell the difference between different intensity levels of a sensory percept) play a fundamental role in in determining the bandwidth of a neuronal communication channel. In the above example with four areas and three distinguishable intensity levels 81 distinct patterns (i.e. permutations) can be generated, perceived and associated with conceptual information to be communicated via the CBI to the individual. Establishing levels that can be differentiated can be executed on consciously perceivable levels and interviewing the individual undergoing the training procedure (open calibration; see Fig. 10) or by employing EEG recordings from the surface of the skull or other means of electrophysiology to record cortical reactions to subcortical white matter stimulation (closed calibration).

The parcellation of the individual cortex is not known a-priori, although scientific methods (e.g. Transcranial Magnetic Stimulation) are known in the art that can be used to generate such individualized maps. In the absence of an individualized map of cortical body representations in e.g. the somatosensory region as distinct as possible axon fiber bundles 120 can be recruited. In this context, *"distinct"* or *"disjunct"* means that every stimulation signal shall recruit axons fiber populations which are not recruited by the other stimulation signals.

An inverse solution can then be calculated to determine which (potentially bipolar and/or biphasic) electrode configuration can recruit the axon fibers most distinctively while avoiding all other fibers as much as possible. In such a step the angle of each axonal structure as it passes the stimulating electrode is considered, as well as factors such as myelinization, axonal diameter and other anatomical properties of relevance for the calculation of electrical conductivity models (e.g. impedance as a function of local scarring or water content in the tissue from edema), consequently optimal stimulation conditions (depending on the actual electrode geometry) for each fiber are calculated and fibers with similar cortical termination zones and geometric proximity are clustered into a channel.

In one embodiment a target axon fiber population is activated via a neuronal stimulation signal having a fixed pulse width and a fixed amplitude (e.g. an electric current in the mA range). The intensity of the elicited sensory percept (e.g. levels 1,2,3 in the above example) can then be instantiated via modulations in the frequency of the stimulation signal.

In greater detail: A brute force approach would generate VTAs within the communication envelope of the electrode from pseudo-random stimulation parameters (or alternatively perform a full walk through the multidimensional parameter space) and
determine the recruited axon fibers and their likely cortical termination zone. After exhausting the parameter space those stimulation settings (e.g. set of signal parameters) which recruit maximally geometrically distant and non-overlapping cortical zones are stored as neuronal communication channels. This last step can even be achieved as simply as clustering the activated cortical voxel 3D centroid coordinates, each cluster would represent the cortical fiber termination zone addressed by a given channel. Naturally non-VTA based methods which actually approximate singular axon fiber activation from neuronal compartment modeling (e.g. performed via the NEURON software) are even better suited. In this case, streamlined axon fibers from tractography (e.g. DTI tractography) can be used as a stand-in for actual axonal fibers and the termination zones are determined as described above.

Figure 10 illustrates how such conceptual / perceptional learning procedure 1000 can be implemented to establish a working neuronal communication system / CBI. In the illustrated example, an individual 1030 was implanted with two neuronal stimulation electrodes 130. The individual 1030 receives a given neuronal stimulation signal 1020 generated by the neuronal communication system 300 connected to the stimulation electrodes 130 via conductor wires. In order to associatively link a certain piece of conceptual information (e.g. a letter, an object, etc.) to the applied neuronal stimulation signal 1020 the individual 1030 is presented with the respective piece of conceptual information via a display screen 1010 and / or a speaker (not shown).

Through means of operant and classical conditioning the associations between a given sensory percept and a piece of conceptual information can be imprinted through training and finally be tested in a validation task (e.g. stimulate a sensory percept and ask the individual to identify the corresponding piece of conceptual information without displaying the corresponding visual and / or auditory cue that was used for establishing the associative link during training.

Via such a classical conditioning paradigm visual cues such as words are depicted on the screen 1010 (preceded and followed by visual Start & Stop cues). During word depiction (message) a characteristic neuronal stimulation signal 1020 is applied via the electrodes 130. After a sufficiently long training run with a large number of repetitions the individual can be tested in a validation run. During validation only, the neuronal stimulation signal 1020 is applied (preceded and followed by visual Start & Stop cues, but without the visual depiction of the word in question) and the individual 1030 is asked to complete a forced choice test to identify the word in question. Above-chance accuracy of the forced choice test can be used to rate success.

In a further training run the stimulation intensities may be reduced such, that no conscious percepts are elicited any longer. Sub-conscious perception of the sensory percepts elicited by the neuronal stimulation signals can still encode the messages communicated to the individual 1030.

Figure 11 shows a block diagram of a calibration and learning procedure for establishing relations between neuronal stimulation signal and corresponding conceptual information to be communicated by a neuronal stimulation system according to an embodiment of the present invention. The personalized communication library is formed through establishing a number of channels (block 1) and the granularity of transmittable sensory percepts (e.g. through calibration, block 2), storing message blocks and associated signal pattern pairs (block 3) and annotating successful interface components (final block) for example by recording post pair-learning phase successful transfer and / or recognition rate performance (decoding of signal patterns by an individual into perceived messages). This can be repeated by encoding a range of messages into the signal patterns (block 4) and transmitting them to the subject (block 5), similar to increasing verbal fluency in a foreign language of a learner by exposing them to a range of books rather than re-reading the same book over and over.

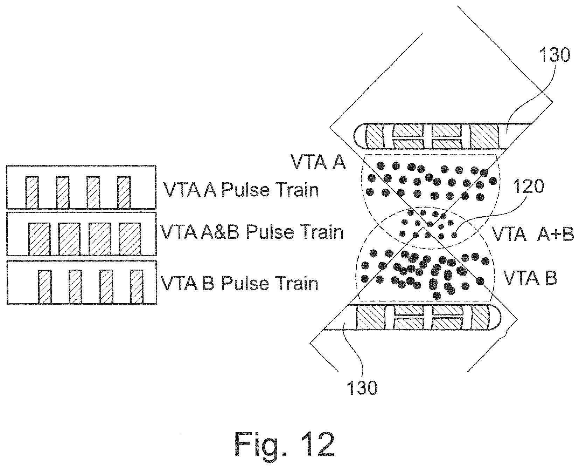

Figure 12 illustrates another embodiment of the present invention, wherein a neuronal stimulation system can be interfaced with at least two neuronal stimulation electrodes 130 for stimulating afferent axons 120 targeting the sensory cortex of an individual. In this embodiment multiple electrodes are implanted in a way that their time-synchronized stimulation patterns can be used to overlap and more selectively stimulate certain axon fiber targets 120.

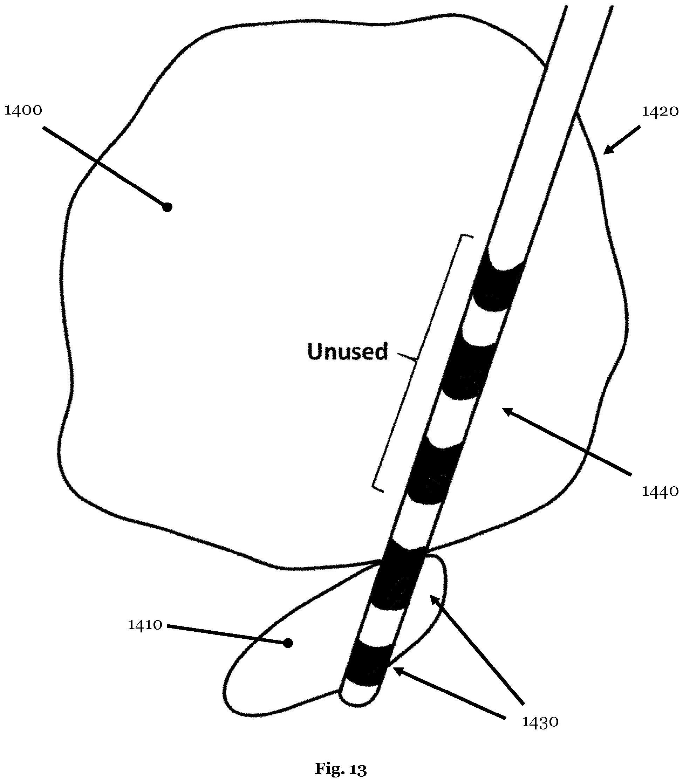

Figure 13 shows an illustration of a multi-contact neuromodulation electrode 1420 adapted for neuromodulation of efferent motor axons. The electrode 1420 can also be used for stimulating afferent axons projecting from the thalamus 1400 to the sensory cortex of an individual via a neuronal stimulation system such as the system 300 of Fig. 3. For example, neuronal stimulation signals may be provided by unused contacts 1440 of the neuromodulation electrode 1420 that was implanted for a therapeutic purpose (e.g. neuromodulation of the subthalamic nucleus 1410 via the therapeutic electric contacts 1430).

For instance, in many cases, a DBS electrode that is used as a neuromodulator, e.g. for treatment of Parkinson, is not always active and / or may comprise independently controllable contacts that are not required for achieving the therapeutic purpose. Thus, the neuromodulation electrode can also be used for applying neuronal stimulation signals provided by a system according to the present invention. For DBS electrodes, specifically, some of the electrode contacts 1440 located outside of the stimulation area of interest are completely unused. However, if implantation in e.g. the subthalamic nucleus 1410 is conducted for the tip contacts 1430 to control, for example, the primary Parkinson symptoms more distal contacts could be used in combination with the above disclosed invention to communicate e.g. a continuous gait biofeedback signal into the brain the patient can utilize to navigate better and/or break free from freezing of gait situations. Such a biofeedback signal can consist of e.g. EMG sensor feedback transmitted to the implant via a smartphone, with the EMG glue-on disposable sensors measuring muscle tension or movement patterns or even simple accelerometer data from a smartwatch.

### Further embodiments relating to behavior modification systems

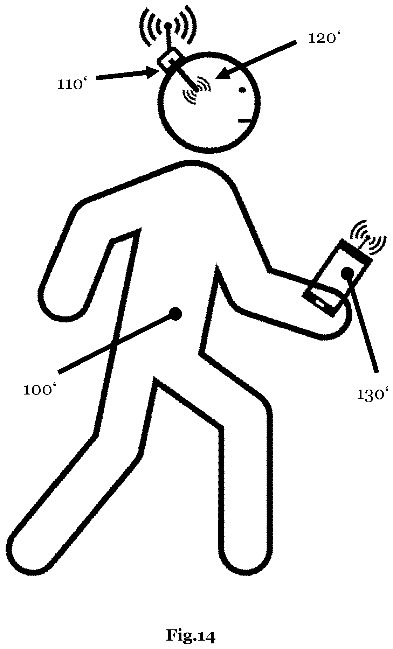

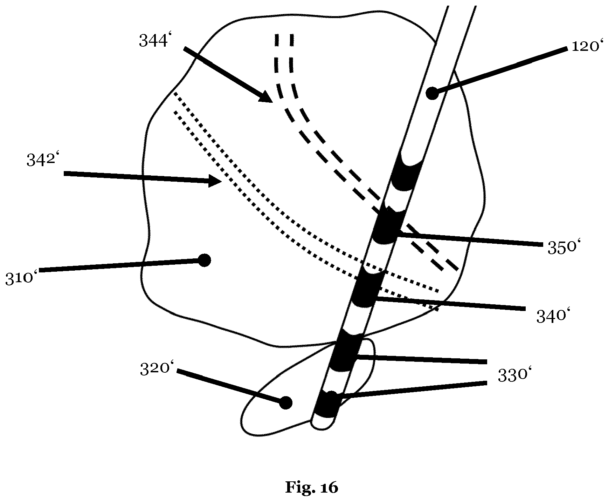

Figure 14 depicts an individual 100', e.g. a PD patient, that has been implanted with a neuronal stimulation electrode 120' such as a DBS electrode that may have multiple independently controllable electric contacts, as illustrated in Fig. 16 below. For instance, the neuronal stimulation electrode 120' may be already implanted into the brain of the individual 100' for the purpose of providing a neuromodulation therapy for certain PD symptoms such as tremor, dystonia and / or rigidity. However, the neuronal stimulation electrode 120' may also be implanted for other purposes such as for the purpose of neuronal communication and /or treatment of other movement impairments and neurological diseases such as epilepsy. Alternatively, the electrode 120' may also be implanted dedicated as an interface for the systems provided by the present invention.

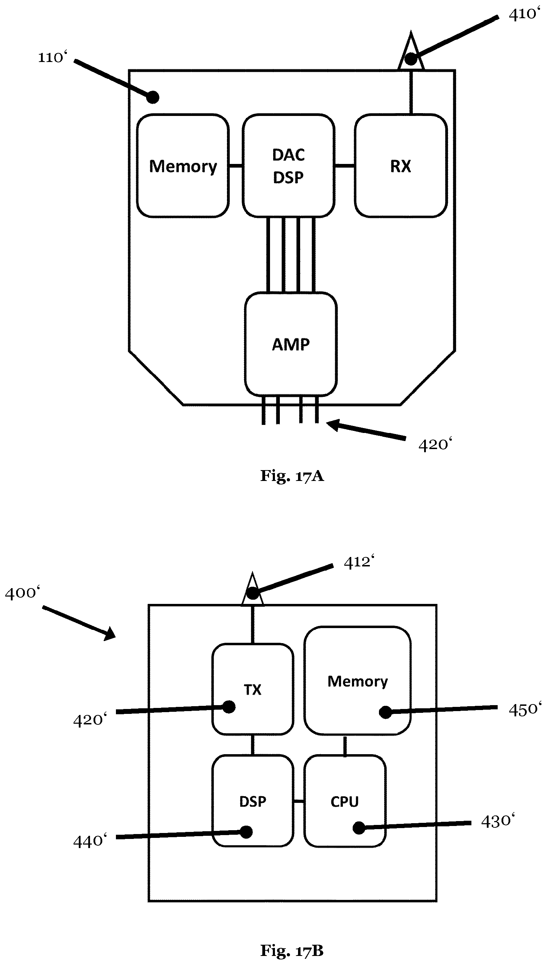

The individual 100' may be further equipped with a neuronal stimulation signal generator device 110' that may be arranged on the head of the individual 100' or somewhere else on or in the vicinity of the body of the individual 100'. The neuronal signal generator 110' may be in wireless communication (e.g. via a Bluetooth or similar wireless interface) with a control device 130', that may be implemented by a smartphone or a similar electronic information processing device. Depending on implementation details the systems provided by the present invention may be implemented by the control device 130', the neuronal signal generator 110, an additional system (such as the system 400' of Fig. 17B) or a combination thereof. For instance, the control device 130', the signal neuronal signal generator device 110' or both may be provided with application specific hardware and / or software modules comprising circuitry and / or software instructions to implement a system according to the present invention.

The control device 130' may provide the individual with a user interface to adjust the neuronal stimulation signals and / or the neuromodulation therapy applied via the signal generator 110' and the neuronal stimulation electrode 120'. For instance, the individual 100' may adjust signal parameters such as a signal frequency, a pulse width, a pulse shape and /or a signal amplitude. For example, the individual may use the control device 130' to select a perceived periodicity of a movement cue provided by a neuronal stimulation signal to the cortex of the individual 100'. For example, if the movement cue is used to provide guidance to the individual 100' during a movement such as walking, the control device 130' may be used to select and set a movement pace associated with the perceived periodicity of the movement cue.

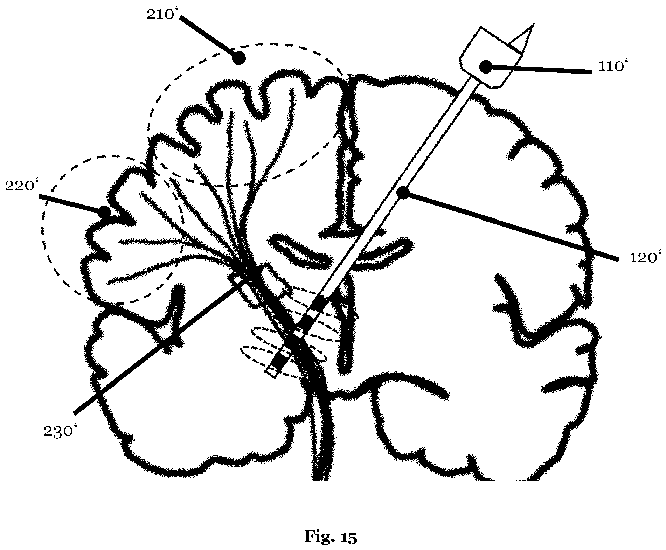

Figure 15 depicts a diagram illustrating a neuronal stimulation electrode 120' for stimulating afferent axons 230' targeting sensory neurons in the cortex of a human brain. The afferent axons 230' may target different areas 210', 220' of the cortex that may be related to different sensory modalities (e.g. touch, temperature sense, vision, hearing, etc.) and / or different body regions (e.g. cochlea, retina, hand, tongue, foot etc.) from which the respective sensory modality is perceived by the respective area of the cortex. For instance, the cortical area 210' may be a somatosensory area of the right foot and the cortical area 220' may be a somatosensory area of the left hand.

The afferent axons 230' are connected via synapses (not shown) with their respective target neurons in the respective sensory area 210', 220'. For instance, the axons 230' may be thalamocortical axons relaying sensory information from the thalamus to the cerebral cortex. The neuronal stimulation electrode 120' may comprises a plurality of independently controllable electric contacts (see Fig. 16 below) that may be arranged in the vicinity of a bundle of afferent axons 230' targeting the sensory areas 220' and 210' of the cerebral cortex.

In the illustrated example, the neuronal stimulation electrode 120' is connected to a neuronal stimulation signal generator 110', which is adapted to apply neuronal stimulation signals to the afferent axons 230', e.g. via independently controllable electric contacts of the neuronal stimulation electrode 120'. In addition, the neuronal stimulation electrode 120' may further comprise a wireless interface for interfacing the signal generator 110' with a neuronal stimulation system which may be adapted to obtain and / or determine the waveform and / or signal parameters (e.g. pulse width, pulse shape, frequency, amplitude, number of pulses etc.) of the neuronal stimulation signal that is generated and applied by the signal generator 110' to the afferent axons 230' via the stimulation electrode 120'.

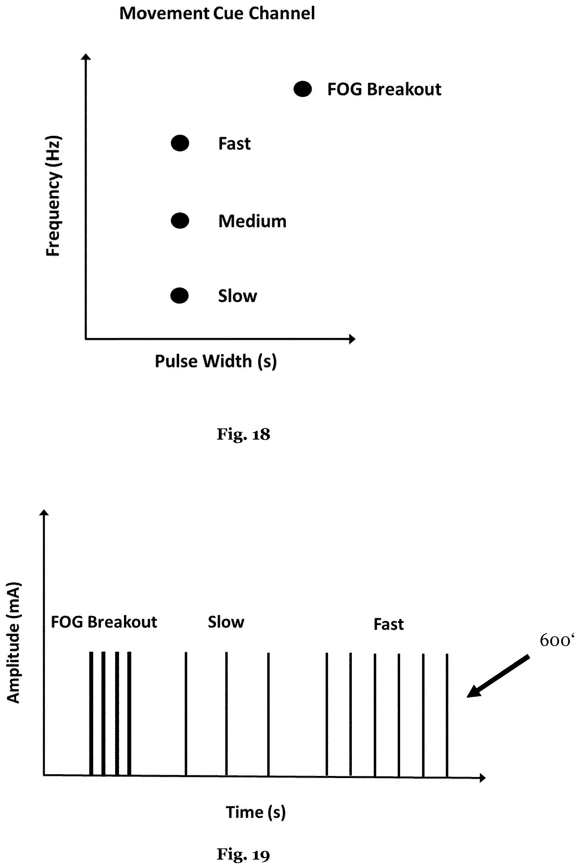

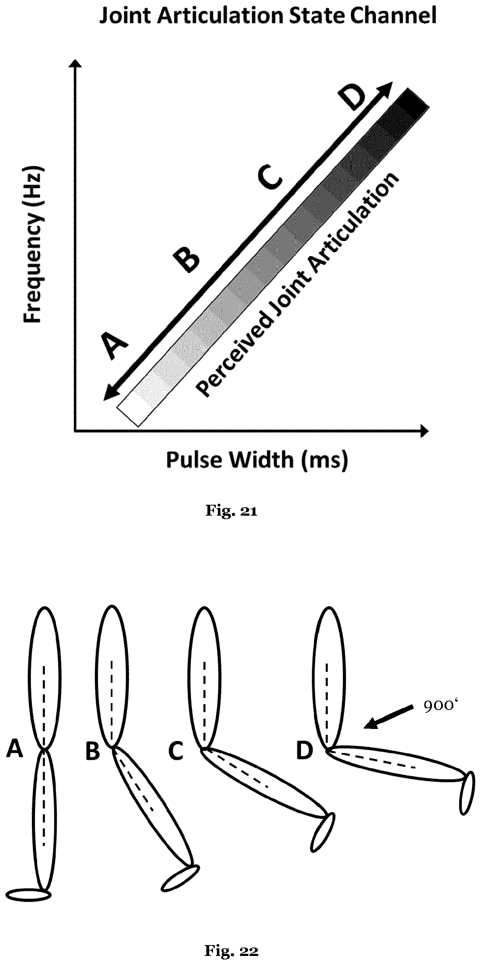

For instance, the neuronal stimulation system provided by the present invention may determine the waveform and / or signal parameters of the neuronal stimulation signal such that a desired sensory percept is elicited in a desired area of the sensory cortex of the individual. In some embodiments of the present invention, the cortex of the individual which is receiving the neuronal stimulation signal (i.e. via afferent action potentials of the stimulated afferent axons 230') may associate the corresponding sensory percept with a movement cue and / or other type of movement related information such as proprioceptive information relating to the body posture of the individual operating the neuronal stimulation system. For example, similar to learning how to understand Morse code, the individual may have previously participated in a learning procedure establishing an associative link between a given sensory percept elicited by a given stimulation signal and a corresponding movement cue (see Figs. 18 and 19 below) or a piece of proprioceptive information (e.g. see Figs. 21 and 22 below) that is to be communicated to the individual via the neuronal stimulation electrode 120'.

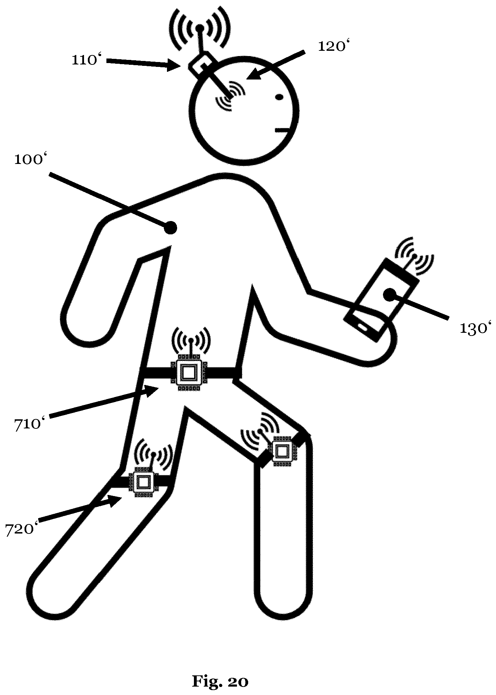

In this approach no nuclei or neuron-rich grey matter are preferably targeted by the neuronal stimulation electrode 120' but preferably the axon-rich white matter of the brain, which contains the information transmitting pathways the brain uses for natural neural communication. In this manner, the present invention provides a white-matter computer-brain-interface (CBI), i.e. a system that generates and provides electrical signals the brain can interpret as meaningful input, e.g. as a rhythmic movement cue or any other type of movement related information such as a commence movement trigger (e.g. a FOG break-out signal) or information about the current body posture of the individual (e.g. proprioceptive information). As discussed in section 3 above, such information may be provided by different types of measurement devices or sensors (see also Fig. 20).

In other embodiments of the present invention, the neuronal stimulation electrode 120', the signal generator 110' and / or the wireless interface may also be part of an integrated neuronal stimulation and /or communication system, e.g. if said components are customized for the intended application. For instance, a neuronal communication system may comprise of specialized communication software running on a multi-purpose information processing device such as a smartphone and a customized assembly of signal generator 110' and stimulation electrode 120' which communicate with the multi-purpose communication device via a wireless interface using conventional wireless data transmission technology such as Wi-Fi, Bluetooth and /or NFC.

In other embodiments of the present invention the neuronal stimulation electrode 120' may be directly connected via wires to a neuronal stimulation system comprising a data processing system and a signal generator similar to the signal generator 110'. In this case a wireless interface is not needed.

Figure 16 depicts of a multi-contact neuromodulation electrode 120' adapted for neuromodulation of the sub-thalamic nucleus 320' via electric contacts 330'. The electrode 120' can also be used for stimulating afferent axons 342', 344' projecting from the thalamus 310' to the sensory cortex of an individual via a neuronal stimulation system according to the present invention. For example, neuronal stimulation signals may be provided by unused contacts 340', 350' of the neuromodulation electrode 120' that was implanted for a therapeutic purpose (e.g. neuromodulation of the subthalamic nucleus 320' via the therapeutic electric contacts 330') different from providing the neuronal stimulation signal to the afferent sensory axons 344', 342'. For instance, the contacts that are not used for neuromodulation of the sub-thalamic nucleus 320' may be used to provide a sensory movement cue and / or proprioceptive information to the cortex of the individual. An example of such a sensory movement cue may be a rhythmic sensory percept elicited by a neuronal stimulation signal applied to the axons 344' targeting a cortex area related to a touch sensation for instance in the left foot.

In many cases, a DBS electrode 120' that is used as a neuromodulator, e.g. for treatment of PD symptoms, is not always active and / or may comprise independently controllable contacts that are not required for achieving the therapeutic purpose. Thus, the neuromodulation electrode can also be used for applying neuronal stimulation signals provided by a system according to the present invention. For DBS electrodes, specifically, some of the electrode contacts located outside of the stimulation area of interest are not used. However, if implantation in e.g. the subthalamic nucleus 320' is conducted for the tip contacts 330' to control, for example, the primary PD symptoms more distal contacts 340', 350' could be used in combination with the above disclosed invention to communicate a movement cue and / or a continuous movement biofeedback signal into the brain the patient can utilize to navigate better and/or break free from FOG.

Figure 17A depicts is a block diagram of a neuronal stimulation signal generator 110' which can be used to apply neuronal stimulation signals to afferent axons 230' via a neuronal stimulation electrode such as the stimulation electrode 110' of Figs. 14 - 16. The neuronal stimulation signal generator 110' may comprise a wireless interface 410' for communicating with a remote neuronal stimulation system (e.g. see Fig. 17B) which may be adapted to obtain, to determine, to select and / or to transmit a waveform and / or signal parameter of the neuronal stimulation signal to the signal generator 110 in order generate a neuronal stimulation signal adapted to elicit a desired sensory percept associated with a movement cue (see Figs. 18 and 19) and / or proprioceptive information (see Fig. 21 and 22).

For instance, the neuronal stimulation signal generator 110' may receive digital data packets specifying a desired neuronal stimulation signal via the wireless interface 410'. Receiver (RX) circuitry may process (e.g. filter, amplify, mix, down-convert to baseband etc.) the received digital data packets and feed the processed digital data packets to a digital signal processor (DSP) with may comprise an integrated digital-to-analog converter (DAC). The DSP then processes the digital data packets to generate one or more neuronal stimulation signals which may then be amplified and applied to a neuronal stimulation electrode such as electrode 120' of Fig. 15 and Fig. 16 by an output amplifier (AMP). For instance, the output AMP may be configured to drive four (or any other number) independently controllable electric contacts 330', 340', 350' of a stimulation electrode such as electrode 120' via the output wires 420'.

In other embodiments, the DSP may receive the digital data packets specifying the neuronal stimulation signal also via a wire-based interface or directly from a collocated processing circuit (e.g. a CPU) which may be adapted to determine the waveform and / or signal parameters of a desired neuronal stimulation signal corresponding to a desired sensory movement cue and / or proprioceptive information to be elicited in the cortex of the to the individual.

Figure 17B depicts a block-type circuit diagram of an exemplary neuronal stimulation system 400' according to an embodiment of the present invention. The neuronal stimulation system 400' may for instance comprise a wireless interface 412' and transmitter (TX) circuitry for communicating (e.g. via Bluetooth or a similar interface) with a neuronal stimulation signal generator such as the generator circuit 110 described above with reference to Fig. 17A. The TX circuitry may be adapted to process (i.e. filter, modulate, mix, amplify, and / or upconvert) digital data packets to be communicated via the wireless interface 412'. The neuronal stimulation system 400 may further comprise a digital signal processor (DSP) operably connected with the TX circuitry and adapted to provide digital data packets specifying the waveform and / or the signal parameters (e.g. frequency, phase, pulse width, pulse amplitude, pulse shape, channel count, etc.) of a desired neuronal stimulation signal to be applied via a neuronal stimulation electrode such as the electrode 120' of Figs. 15 and 16 and via a neuronal stimulation signal generator such as signal generator 110' of Fig. 17A.

The neuronal stimulation system 400' may further comprise general data processing circuitry such as a CPU operably connected to the DSP and at least one digital memory device operably connected to the CPU. The CPU 320' and the memory may interact to determine a desired neuronal stimulation signal corresponding to a desired sensory percept such as the desired movement cue and / or the desired proprioceptive information to be communicated to the cortex of the individual.

For instance, the memory may contain a personalized communication library for the individual, the library storing relations between a plurality of movement cues and / or perceptive information blocks and a plurality of corresponding neuronal stimulation signals.

Such a stimulation library can be calibrated for each individual through neuroimaging and / or individualized testing of the individual. Neuroimaging may first be used to identify theoretically possible ranges of activation for an individual stimulation electrode while individualized testing determines which points in the parameter space of stimulation signal parameters (for details see Fig. 18 and Fig. 22 below) can be perceived and decoded by the cortex of the individual. It should be emphasized that conscious individualized testing of an individual is merely one specific example how to generate the individualized relations stored in the memory. In other embodiments such relations may also be obtained from unconscious patients, e.g. through the non-invasive observation of corresponding functional MRI responses on the somatosensory cortex or EEG recordings.

Further, once or while the communication library (i.e. the plurality of relations stored in the memory) is established or is being established for an individual a specific training procedure can be executed (again not necessarily in a conscious individual). As long as the cortex of the individual responds to classical conditioning, pair learning can be executed. In the context of the present invention, such a pair consists of a given sensory percept corresponding to a given neuronal stimulation signal and a movement cue and /or a piece of proprioceptive information to be associated with said given sensory percept and the corresponding neuronal stimulation signal.

Importantly, the type of information to be conveyed via the neuronal stimulation system 400' whether it is a movement cue, a FOG breakout signal or a piece of proprioceptive information can be chosen freely. Any information or message which can be broken down into message blocks (i.e. pieces of conceptual information that can be decoded by the cortex of an individual) can be transmitted. This includes continuous signals such as signals needed for e.g. an artificial balance, orientation signals or other sensor measurement signals.

Learning paradigms for continuous signals deviate from classical conditioning, since they involve more interactive training scenarios where utilization of the signal is a relevant success factor (e.g. orientation in an artificial virtual environment using the input signal). Continuous signals (e.g. intensity) also deviate from signal configurations for messages containing sequentially delivered message blocks. In the case of continuous signals, intensity might be coded via either pulse width or frequency variations (or combinations of the two; see Fig. 22 below), while not varying the location and target areas in the sensory cortex targeted by the recruited axon fibers.

Figure 18 and 19 illustrates how embodiments of the present invention can be used to establish a sensory movement cueing channel to the to the cortex of an individual and to use said cueing channel to provide a periodic movement cue and a FOG break-out signal that may be used for behavior modification, e.g. to support the individual during a walking task.

For instance, three different walking paces (e.g. 1 step per second, 0,5 steps per second, 2 steps per second) may be encoded by providing a pulse train signal via a neuronal stimulation interface and system as discussed above. Such a pulse train (being characterized by signal parameters such as pulse width, pulse frequency, pulse shape and / or pulse amplitude) may elicit a periodic / rhythmic sensory percept in the targeted area of the sensory cortex of the individual. For instance, such a pulse train signal may be configured to elicit a periodically appearing tough sensation in the palm of the right hand or in a leg of the individual. Similar to an auditory movement cue provided to the individual via earphones such a neuronal movement cue may help the individual to walk at a constant pace and without experiencing a FOG period. Moreover, the same neuronal communication channel can also be used to communicate a FOG breakout signal to the individual. For instance, the FOG breakout signal may be encoded by choosing a different combination of pulse train parameters such as a combination of a larger pulse frequency and a larger pulse width as indicted in Fig. 18.

Fig. 19 illustrates a typical use scenario of a neuronal stimulation system provided by the present invention. When occurrence of a FOG event has been determined (either by the neuronal stimulation system itself, a human supervisor or a control device associated with the individual performing the walking task) the neuronal stimulation system may obtain the FOG breakout signal (e.g. from its memory or via a wireless communication interface) and transmits it to an electric contact of a neuronal stimulation electrode such as the DBS electrode illustrated in Figs. 15 and 16 to suppress the FOG event.

After a FOG event has been suppressed the neuronal stimulation system can switch into a pacemaker operation mode and may apply a slow periodic movement cue to help the individual to resume normal walking. After the individual has resumed slow walking, he could provide a user input to the neuronal stimulation system indicating the intention to switch from the slow movement cue to a faster one.

Such user input may for example be provided via a control device such as a smartphone or via a neuronal excitation measurement equipment recording a neuronal excitation pattern corresponding to a motor intent of the individual. For instance, such a neuronal excitation measurement equipment may involve recoding from the contacts of the same neuronal electrode that is also used for applying the neuronal stimulation signal (e.g. in the form of Local Field Potentials). In some embodiments the system may also comprise an EEG device, a sub-dural electrode array and / or a transcranial excitation measurement device.

Such neuronal excitation measurement equipment may be used to provide the individual with an essentially closed loop stimulation system, wherein measurements motor related neuronal excitation patterns directly affect how the neuronal stimulation system is operating.

Figure 20 depicts an individual 100', e.g. a PD patient, having been implanted with a neuronal stimulation electrode 120' such as a DBS electrode that may have multiple independently controllable electric contacts, as illustrated in Fig. 16. In addition to the devices discussed above with reference to Fig. 14 the individual 100' may further be equipped with sensors 710' and 720' that are configured to obtain information on the body posture of the individual 100'. For instance, sensor 710' may be configured to measure the balance of the body of the individual 100' while the sensors 720' may be configured to measure the articulation state / flexing angle of the knee joints of the individual 100'.

The sensors 710', 720' may be in wireless communication with the neuronal signal generator 110', the control device 130' and / or a neuronal stimulation system similar to the one discussed above with reference to Fig. 17B.

The measurement signals (providing information about the body posture of the individual 100') may be transmitted by the sensors 710' and 720' to the processing means of a neuronal stimulation system. The system may then determine, based on the obtained information, a neuronal stimulation signal to be applied to at least one afferent axon targeting at least one sensory neuron in the cortex of the individual, wherein the determined neuronal stimulation signal corresponds to proprioceptive information that is communicated to the individual.

For instance, as illustrated in Fig. 21 and Fig. 22 neuronal stimulation system may use the measurements of the knee joint sensors 720' to determine a neuronal stimulation signal that is adapted to communicate the articulation state of the knee joint to the cortex of the individual. As shown in Fig. 21, the articulation state of the knee joint may be encoded by a combination of signal parameters such as pulse width and pulse frequency of a pulse train signal. In the example shown in Fig. 21 a low frequency pulse train having a short pulse width (A) corresponds to a knee joint that is essentially fully stretched out (A) whereas a high frequency pulse train having a long pulse width (D) corresponds to a knee joint that is almost fully bent (D).

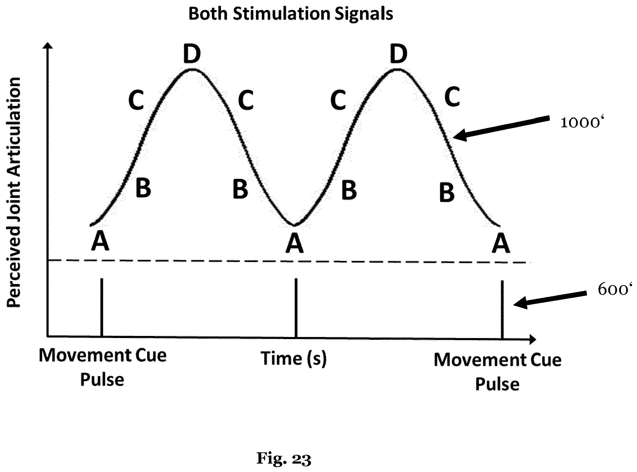

Figure 23 illustrates how the movement cueing channel of Figs. 18 and 19 and the proprioceptive information channel of Fig. 21 and 22 can be combined to improve the performance of a neuronal stimulation system intended for treatment of movement impairments. In the illustrated example a neuronal stimulation signal that is providing the joint articulation state information (e.g. the proprioceptive information channel) is applied quasi-continuously while the individual performs a movement (e.g. walking, dancing etc.) paced by the movement cue signal. If the individual walks synchronized with the movement cue signal every time a movement cue pulse is perceived by the individual the joint articulation signal communicates a fully stretched state (A) of the knee joint to the individual. Between two movement cue pulses the knee joint of the individual first bends (A-B-C-D) and then stretches out again (D-C-B-A).

Compared to systems that only provide movement cues a system providing both, a movement cue and proprioceptive information together to the cortex of an individual substantially improves the movement performance of the individual because synchronizing the movement of the individual with an external movement cue is much easier if proprioceptive information is provided as feedback to the brain that indicates the phase of the movement relative to the timing of the movement cue.

### Further embodiments relating to device status signaling

In the following, some exemplary embodiments of the present invention are described in more detail, with reference to neuronal stimulation and / or communication systems that can be interfaced with neuronal stimulation electrodes such as deep brain stimulation (DBS) electrodes. However, the systems provided by the present invention can also be used with different neuronal stimulation means (e.g. opto-neuronal, chemical-neuronal) that are capable to stimulate the sensory cortex of an individual e.g. via stimulating afferent axons targeting the sensory cortex.

While specific feature combinations are described in the following with respect to the exemplary embodiments of the present invention, it is to be understood that the disclosure is not limited to such embodiments. In other words, not all features have to be present for realizing the invention, and the embodiments may be modified by combining certain features of one embodiment with one or more features of another embodiment. Specifically, the skilled person will understand that features, components and / or functional elements of one embodiment can be combined with technically compatible features, components and / or functional elements of any other embodiment of the present invention.

Figure 14 depicts an individual 100', e.g. a PD patient, that has been implanted with a neuronal stimulation electrode 120' such as a DBS electrode that may have multiple independently controllable electric contacts, as illustrated in Fig. 16. For instance, the neuronal stimulation electrode 120' may be already implanted into the brain of the individual 100' for the purpose of providing a neuromodulation therapy for certain PD symptoms such as tremor, dystonia and / or rigidity. However, the neuronal stimulation electrode 120' may also be implanted for other purposes such as for the purpose of neuronal communication and /or treatment of other movement impairments and neurological diseases such as Alzheimer's disease, epilepsy, depression, etc. Alternatively, the electrode 120' may also be implanted as a dedicated interface for the systems provided by the present invention.

The individual 100' may be further equipped with a neuronal stimulation device 110' that may be arranged on the head of the individual 100' or somewhere else on or in the vicinity of the body of the individual 100'. The neuronal stimulation device 110' may be in wireless communication (e.g. via a Bluetooth, WI-FI or similar wireless interface) with a control device 130', that may be implemented by a smartphone or a similar electronic information processing device. Depending on implementation details the systems provided by the present invention may be implemented via application specific hardware and / or software modules comprising circuitry and / or software instructions to implement a system according to the present invention.

The control device 130' may provide the individual with a user interface to adjust the neuronal stimulation signals and /or the neuromodulation therapy parameters applied via the neuronal stimulation device 110' and the neuronal stimulation electrode 120'. The control device 130' may also provide connectivity to a packet based wireless large area network such as an LTE or 5G network. For instance, the individual 100' may use the control device 130' to adjust signal parameters such as a signal frequency, a pulse width, a pulse shape and /or a signal amplitude as well as for retrieving data from the internet.

Figure 15 depicts a diagram illustrating a neuronal stimulation electrode 120' for stimulating afferent axons 230' targeting sensory neurons in the cortex of a human brain. The afferent axons 230' may target different areas 210', 220' of the cortex that may be related to different sensory modalities (e.g. touch, temperature sense, vision, hearing, etc.) and / or different body regions (e.g. cochlea, retina, hand, tongue, foot etc.) from which the respective sensory modality is perceived by the respective area of the cortex. For instance, the cortical area 210' may be a somatosensory area of the right foot and the cortical area 220' may be a somatosensory area of the left hand.

The afferent axons 230' are connected via synapses (not shown) with their respective target neurons in the respective sensory area 210', 220'. For instance, the axons 230' may be thalamocortical axons relaying sensory information from the thalamus to the cerebral cortex. The neuronal stimulation electrode 120' may comprises a plurality of independently controllable electric contacts (see Fig. 16) that may be arranged in the vicinity of a bundle of afferent axons 230' targeting the sensory areas 220' and 210' of the cerebral cortex.

In the illustrated example, the neuronal stimulation electrode 120' is connected to a neuronal stimulation device 110', which is adapted to apply neuronal stimulation signals to brain areas associated with certain neurophysiological symptoms and / or to the afferent axons 230', e.g. via independently controllable electric contacts of the neuronal stimulation electrode 120'. In addition, the neuronal stimulation electrode 120 may further comprise a wireless interface for interfacing the neuronal stimulation device 110 with other systems that may which may be adapted to obtain and / or determine the waveform and / or signal parameters (e.g. pulse width, pulse shape, frequency, amplitude, number of pulses etc.) of the neuronal stimulation signal that is generated and applied by the neuronal stimulation device 110' to the afferent axons 230' via the stimulation electrode 120'.

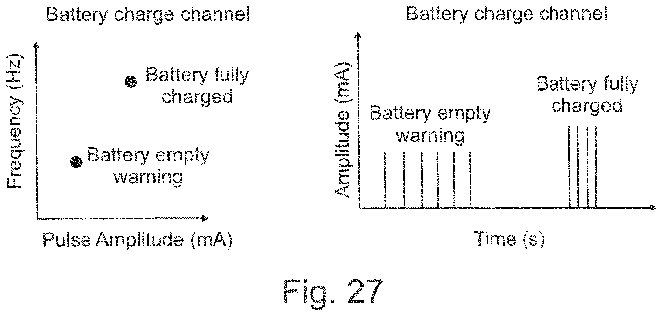

For instance, the neuronal stimulation system provided by the present invention may determine the waveform and / or signal parameters of the neuronal stimulation signal such that a desired sensory percept is elicited in a desired area of the sensory cortex of the individual. In some embodiments of the present invention, the cortex of the individual which is receiving the neuronal stimulation signal (i.e. via afferent action potentials of the stimulated afferent axons 230') may associate the corresponding sensory percept with several types of device status information such as a charging level of a battery, a hardware fault status, a communication status etc. For example, similar to learning how to understand Morse code, the individual may have previously participated in a learning procedure establishing an associative link between a given sensory percept elicited by a given neuronal stimulation signal and a corresponding device status (e.g. see Figs. 27, 30, 32 and 33 below) that is to be communicated to the individual via the neuronal stimulation electrode 120'.

In this approach no nuclei or neuron-rich grey matter are preferably targeted by the neuronal stimulation electrode 120' but preferably the axon-rich white matter of the brain, which contains the information transmitting pathways the brain uses for natural neural communication. In this manner, the present invention provides a white-matter computer-brain-interface (CBI), i.e. a system that generates and provides electrical signals the brain can interpret as meaningful input, e.g. as a charging level of the battery of the device 110'. As discussed in section 3 above, such information may be provided by different types of measurement devices or sensors.

In other embodiments of the present invention, the neuronal stimulation electrode 120', the neuronal stimulation device 110' and / or the wireless interface may also be part of an integrated neuronal stimulation and /or communication system, e.g. if said components are customized for the intended application. For instance, a neuronal communication system may comprise of specialized communication software running on a multi-purpose information processing device such as a smartphone and a customized assembly of neuronal stimulation device 110' and stimulation electrode 120' which communicate with the multi-purpose communication device via a wireless interface using conventional wireless data transmission technology such as Wi-Fi, Bluetooth and /or NFC.

In other embodiments of the present invention the neuronal stimulation electrode 120' may be directly connected via wires to a neuronal stimulation system comprising a data processing system and a neuronal stimulation device similar to the neuronal stimulation device 110'. In this case a wireless interface is not needed.

Figure 16 depicts a multi-contact neuromodulation electrode 120' e.g. adapted for neuromodulation of the sub-thalamic nucleus 320' via electric contacts 330'. The electrode 120' can also be used for stimulating afferent axons 342', 344' projecting from the thalamus 310' to the sensory cortex of an individual via a neuronal stimulation system according to the present invention. For example, neuronal stimulation signals may be provided by unused contacts 340', 350' of the neuromodulation electrode 120 that was implanted for a therapeutic purpose (e.g. neuromodulation of the subthalamic nucleus 320 via the therapeutic electric contacts 330') different from providing the neuronal stimulation signal to the afferent sensory axons 344', 342'. For instance, the contacts that are not used for neuromodulation of the sub-thalamic nucleus 320' may be used to provide different kinds of device status information to the cortex of the individual. For example, such device status information may be signaled via a rhythmic sensory percept elicited by a neuronal stimulation signal applied to the axons 344' targeting a cortex area related to a touch sensation for instance in the left foot or the right hand.

In many cases, a DBS electrode 120' that is used as a neuromodulator, e.g. for treatment of symptoms of PD etc., is not always active and / or may comprise independently controllable contacts that are not required for achieving the therapeutic purpose. Thus, the neuromodulation electrode can also be used for applying neuronal stimulation signals provided by a system according to the present invention. For DBS electrodes, specifically, some of the electrode contacts located outside of the stimulation area of interest are not used. However, if implantation in e.g. the subthalamic nucleus 320' is conducted for the tip contacts 330' to control, for example, the primary PD symptoms more distal contacts 340', 350' could be used in combination with the above disclosed invention to communicate a device status information signal directly into the brain of the patient.

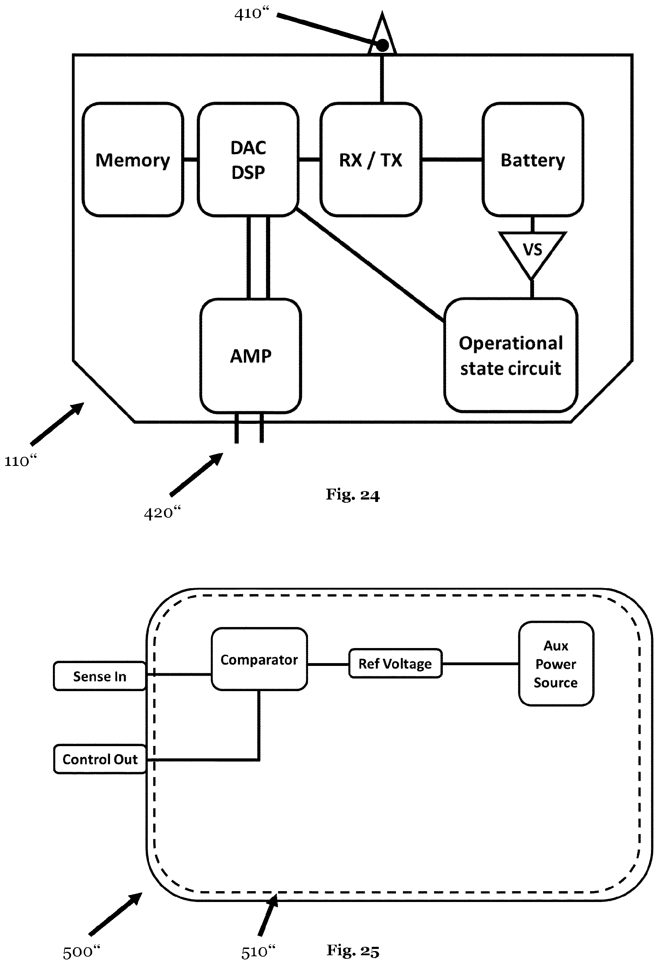

Figure 24 depicts is a block diagram of a neuronal stimulation device 110" which can be used to apply neuronal stimulation signals to afferent axons 230" via a neuronal stimulation electrode such as the stimulation electrode 120' of Figs. 14 - 16. The neuronal stimulation device 110" may comprise a wireless interface 410" for communicating with a remote control device 130" and / or a remote signal generation device which may be adapted to obtain, to determine, to select and / or to transmit a waveform and / or signal parameter of the neuronal stimulation signal to the neuronal stimulation device 110".

For instance, the neuronal stimulation neuronal stimulation device 110" may receive digital data packets specifying a desired neuronal therapy or communication signal via the wireless interface 410". Receiver (RX) circuitry may process (e.g. filter, amplify, mix, down-convert to baseband etc.) the received digital data packets specifying the waveform and / or the signal parameters (e.g. frequency, phase, pulse width, pulse amplitude, pulse shape, channel count, etc.) and feed the processed digital data packets to a digital signal processor (DSP) with may comprise an integrated digital-to-analog converter (DAC). The DSP then processes the digital data packets to generate one or more neuronal stimulation signals which may then be amplified and applied to a neuronal stimulation electrode such as electrode 120' of Fig. 15 and Fig. 16 by an output amplifier (AMP). For instance, the output AMP may be configured to drive four (or any other number) independently controllable electric contacts 330", 340", 350" of a stimulation electrode such as electrode 120" via the output wires 420".

In other embodiments, the DSP may receive the digital data packets specifying the neuronal stimulation signal also via a wire-based interface or directly from a collocated processing circuit (e.g. a CPU) which may be adapted to determine the waveform and / or signal parameters of a desired therapy or communication signal corresponding.

The neuronal stimulation apparatus 110" may further comprise an operational state circuit configured to monitor one or more types of device status information. For instance, the operational state circuit may be configured to monitor a voltage level of the battery of the device 110" via a voltage sensor (VS). The operational state circuit may be further configured for determining a battery charge level based on the output of the voltage sensor.

The operational state circuit may be further configured to provide an input signal to the DSP instructing the DSP to obtain, to determine, to select and / or to generate a neuronal stimulation signal that is to applied to a neuronal stimulation electrode via the output amplifier AMP and the output wires 420". For instance, in response to the input signal form the operational state circuit, the DPS may obtain signal parameters or the waveform of a neuronal stimulation signal from a memory device, wherein the neuronal stimulation signal corresponds to a device status information associated with the monitored battery charge level. For instance, the neuronal stimulation signal may correspond to a low-battery warning signal to be communicated to the individual using the neuronal stimulation device 110" (see Fig. 27 below).

The neuronal stimulation device 110" may further comprise at least one memory device operably connected to the DSP. The DSP and the memory may interact to determine a desired neuronal stimulation signal corresponding to a desired sensory percept such as the desired device status information to be communicated to the cortex of the individual.

For instance, the memory may contain a personalized communication library for the individual, the library storing relations between a plurality of device statuses and a plurality of corresponding neuronal stimulation signals.

Such a stimulation library can be calibrated for each individual through neuroimaging and / or individualized testing of the individual. Neuroimaging may first be used to identify theoretically possible ranges of activation for an individual stimulation electrode while individualized testing determines which points in the parameter space of stimulation signal parameters (for details see Figs. 27, 20, 32

and 33 below) can be perceived and decoded by the cortex of the individual. It should be emphasized that conscious individualized testing of an individual is merely one specific example how to generate the individualized relations stored in the memory. In other embodiments such relations may also be obtained from unconscious patients, e.g. through the non-invasive observation of corresponding functional MRI responses on the somatosensory cortex or EEG recordings.

Further, once or while the communication library (i.e. the plurality of relations stored in the memory) is established or is being established for an individual a specific training procedure can be executed (again not necessarily in a conscious individual). As long as the cortex of the individual responds to classical conditioning, pair learning can be executed. In the context of the present invention, such a pair consists of a given sensory percept corresponding to a given neuronal stimulation signal and a device status to be associated with said given sensory percept and the corresponding neuronal stimulation signal.

Importantly, the type of information to be conveyed via the neuronal stimulation device 110" whether it is a battery status signal, a communication status signal or a hardware fault status signal etc. can be chosen freely. Any information or message which can be broken down into message blocks (i.e. pieces of conceptual information that can be decoded by the cortex of an individual) can be transmitted. This includes continuous signals such as signals needed for a (quasi-)continuous battery charge indication (see Fig. 32 below) or other sensor measurement signals.

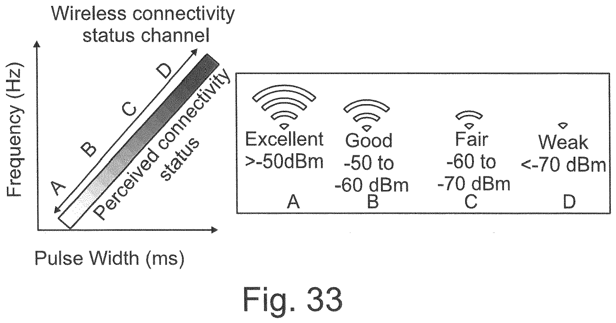

Learning paradigms for continuous signals deviate from classical conditioning, since they involve more interactive training scenarios where utilization of the signal is a relevant success factor (e.g. orientation in an artificial virtual environment using the input signal). Continuous signals (e.g. intensity) also deviate from signal configurations for messages containing sequentially delivered message blocks. In the case of continuous signals, intensity might be coded via either pulse width or frequency variations (or combinations of the two; see Figs. 32 and 33 below), while not varying the location and target areas in the sensory cortex targeted by the recruited axon fibers.

Figure 25 depicts a block-type circuit diagram of an exemplary operational state circuit 500". The operational state circuit may comprise an input terminal (Sense In) e.g. connected to the output of a voltage sensor VS (see Fig. 24) and an output terminal (Control Out) e.g. connected to an input of a DSP (see Fig. 24). The operational state circuit may further comprise an auxiliary power source and a comparator circuit (e.g. a standard OP-AMP circuit) that compares the input signal being applied to Sense In to a reference voltage (Ref voltage). If the input voltage signal falls below (above) the reference voltage the comparator produces an output signal that is applied via Control Out to the DSP. In response to the input signal from the operational state circuit 500", the DPS may then obtain a waveform (or signal parameters specifying a waveform) of a neuronal stimulation signal such as a pulse train signal from memory, wherein the neuronal stimulation signal corresponds to a device status information associated with the monitored battery charge level. For instance, the neuronal stimulation signal may correspond to a low-battery warning signal to be communicated to the individual using the neuronal stimulation device 110".

The operational state circuit 500" may further comprise magnetic shielding equipment 510 protecting the circuit elements of the operational state circuit, thereby increasing the reliability and fail-safe operation of the operational state circuit 500".

Figure 26 depicts is a block diagram of a neuronal stimulation device 600" that is similar to the neuronal stimulation device of Fig. 24 discussed above. In addition to the circuit components discussed above, the neuronal stimulation device 600" comprises a switch or multiplexing circuitry (Switch / MUX) that allows to apply a neuronal stimulation signal generated by the operational state circuit (which comprises an auxiliary signal amplifier) to the output wires 610" that connect to a neuronal stimulation electrode, such as the multi-contact electrode discussed above with reference to Figs. 15 and 16.

In the example of Fig. 26 the operational state circuit also comprises means to generate a neuronal stimulation signal corresponding to the monitored battery charge status. In this way, the battery charge status signal can even be transmitted to the brain of the individual without involving the primary signal processing circuitry of the device 600" that may not be functional anymore. Similar to the operational state circuit of Fig. 25 also the device 600" may further comprise magnetic shielding equipment protecting the voltage sensor (VS) the operational state circuit and / or the switch or multiplexing circuit.

Figure 27 illustrates how embodiments of the present invention can be used to establish a binary battery charge status channel to the to the cortex of an individual and to use said battery charge status channel to provide a indicate to the individual to recharge the battery.

For instance, two different battery charge status signals may be encoded by providing a pulse train signal via a neuronal stimulation interface and system as discussed above. Such a pulse train (being characterized by signal parameters such as pulse width, pulse frequency, pulse shape and / or pulse amplitude) may elicit a periodic / rhythmic sensory percept in the targeted area of the sensory cortex of the individual. For instance, such a pulse train signal may be configured to elicit a periodically appearing tough sensation in the palm of the right hand or in a leg of the individual. For instance, a battery empty warning signal may be encoded by choosing a certain combination of pulse train parameters such as a combination of a relatively lower pulse frequency and a relatively small pulse amplitude as indicted in Fig. 27. Similarly, a higher pulse frequency and larger amplitude may be associated with a signal indicating that the battery is fully charged.

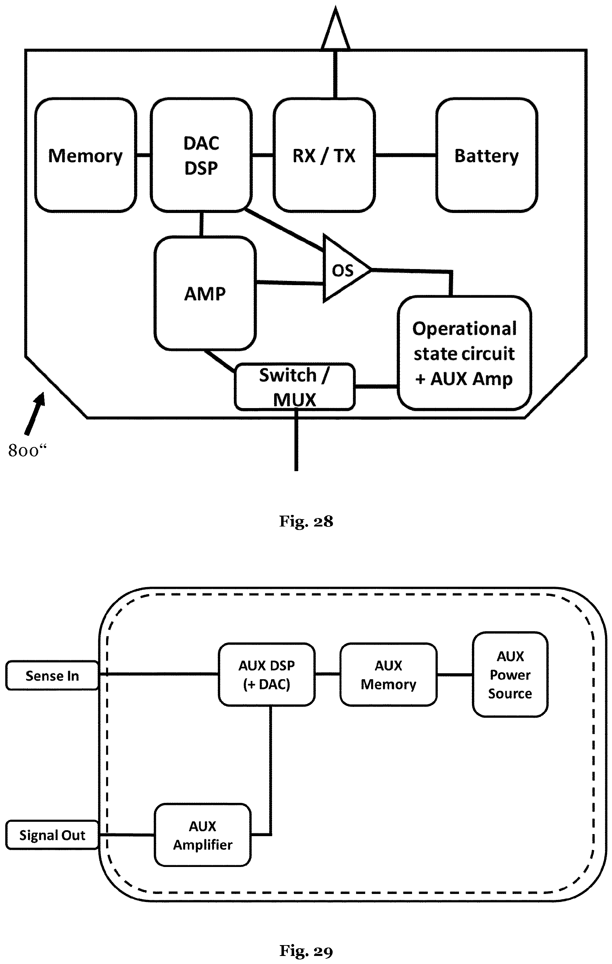

Figure 28 illustrates a further example of a neuronal stimulation device being equipped with a system according to the present invention. Similar to the neuronal stimulation devices 110" and 600" of Figs. 24 and 26 the device 800" depicted in Fig. 28 comprises an operational state circuit adapted to monitor one or more types of device status information. For instance, the operational state circuit may receive input signals from one or more operational state sensors (OS) monitoring whether certain primary circuit elements (such as the DSP of the output amplifier) of the device 800" operate within specified operation parameter ranges. For instance, the OS may monitor a temperature of the DSP and /or of the AMP to inform the individual when these components may overheat.

Figure 29 depicts another example of an operational state circuit 900" that may be used in or with a system according to the present invention. The operational state circuit 900" comprises all circuity necessary for generating and transmitting neuronal stimulation signals corresponding to various kinds of device status information. For instance, an auxiliary DSP (AUX DSP) may receive various kinds of input signal via Sense In such as a signal indicating that the primary DPS has been switches off due to overheating. In this case, the DSP retrieves a waveform or signal parameters specifying a waveform of a neuronal stimulation signal from an auxiliary memory (AUX Memory) device, the retrieved neuronal stimulation signal indicating a fault state of the neuronal stimulation device to the individual. In this way, the individual can even be informed of a critical fault of the neuronal stimulation device, even if said critical fault would otherwise prevent the neuronal stimulation device to generate neuronal stimulation signals.

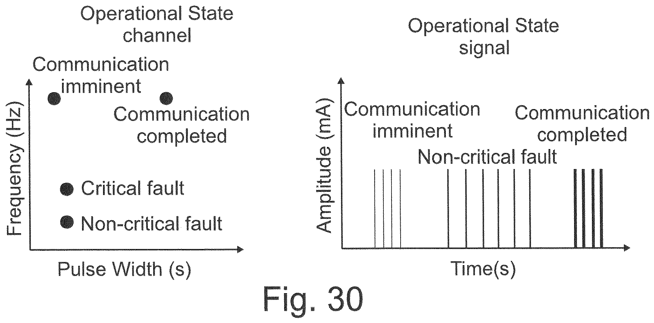

Figure 30 illustrates how embodiments of the present invention can be used to establish a perceptive communication channel to the to the cortex of an individual for signaling both, a communication status and a fault status of a neuronal stimulation device.

A first set of pulse frequencies and pulse widths may be used to signal to the individual that communication of the actual device status information is imminent (e.g. will commence soon) or completed. In this way it can be ensured that the individual using such a neuronal stimulation device focusses his attention to the device status information to be communicated. A second set of set of pulse frequencies and pulse widths may then be used to signal the actual device status information (or any other kind of information to be communicated to the individual).

Figure 31 shows another example of a neuronal stimulation device that is similar to the devices discussed above for Figs. 24, 26 and 28. In this example, the operational state circuit is adapted to receive monitoring signals from a neuronal signal sensor (NS) that monitors the primary neurostimulation signals outputted via the output vires 1110" (e.g. a neuromodulation signal for treatment of PD). For instance, the NS may be connected to a reference electrode attached to the skin or the skull (e.g. sub- or supra-dural) of the individual using the device 1100". The NS may generate a voltage signal that is related to the neuronal stimulation signals applied via wires 1110". The operational state circuit may be configured to use the voltage signal generated by the NS to determine whether the signal parameters of the primary neuronal stimulation signals are still within specified parameter ranges required for proper functioning of the device 1100". For instance, if the voltage signal of the NS indicates that the signal amplitude and / or frequency of the primary neuronal stimulation signal is too low for achieving the desired therapeutic purpose, the operational state circuit may cause the DSP to generate a neuronal stimulation signal to be applied via wires 1110" to a neuronal stimulation electrode indicating to the treated individual that maintenance of the device 1100" is required.

Figure 32 illustrates how a (quasi-) continuous neuronal communication channel can be implemented that may be used for signaling the charging level of a battery directly to the brain of an individual.

The measurement signal of a battery voltage sensor may be used to determine a neuronal stimulation signal to be applied to at least one afferent axon targeting at least one sensory neuron in the cortex of the individual, wherein the determined neuronal stimulation signal corresponds to the battery charge level that is communicated to the individual.

As shown in Fig. 32, battery charge level may be encoded by a combination of signal parameters such as pulse width and pulse frequency of a pulse train signal. In the example shown in Fig. 32 a low frequency pulse train having a short pulse width (A) corresponds to an almost empty battery (A) whereas a high frequency pulse train having a long pulse width (D) corresponds to a fully charged battery (D).

Figure 32 illustrates how a (quasi-) continuous neuronal communication channel can be implemented that may be used for signaling the signal quality of a wireless communication interface of a neuronal stimulation device.

For instance, the measurement signal of a signal strength sensor may be used to determine a neuronal stimulation signal to be applied to at least one afferent axon targeting at least one sensory neuron in the cortex of the individual, wherein the determined neuronal stimulation signal corresponds to the signal strength of a wireless reference signal level that is communicated to the individual.

As shown in Fig. 32, the wireless signal strength may be encoded by a combination of signal parameters such as pulse width and pulse frequency of a pulse train signal. In the example shown in Fig. 32 a low frequency pulse train having a short pulse width (A) corresponds to a strong signal (A) whereas a high frequency pulse train having a long pulse width (D) corresponds to a very weak wireless signal quality (D).

## Claims

1. System (300, 140) for communicating conceptual information (510, 610, 710) to an individual, comprising:
data storing means (330) storing relations (332), specific for the individual, between a plurality of sensory percepts, each associated with corresponding conceptual information (510, 610, 710) and a plurality of corresponding neuronal stimulation signals (500, 600, 700);
wherein the stored relations link each sensory percept, elicited by the corresponding neuronal stimulation signal with the corresponding conceptual information;
means for selecting (320) at least one neuronal stimulation signal (500, 600, 700) to be applied to at least one afferent axon (120) targeting at least one sensory neuron in a sensory cortex area (100, 110, 112) of the individual, wherein the means for selecting (310) the at least one neuronal stimulation signal (500, 600, 700) comprise means for accessing the data storing means (330);
wherein the selected at least one neuronal stimulation signal (500, 600, 700) corresponds to the conceptual information (510, 610, 710) to be communicated to the individual; and
means for transmitting (340, 350, 310,) the selected at least one neuronal stimulation signal (500, 600, 700) to a neuronal stimulation means (130) of the individual,
wherein the selected at least one neuronal stimulation signal (500, 600, 700) is adapted to elicit a sequence of action potentials in the at least one afferent axon (120) and thereby to evoke a corresponding sensory percept in the targeted sensory cortex area (100, 110, 112) of the individual to communicate the conceptual information associated with the selected sensory percept to the individual,
wherein the stored relations are based at least in part on conceptual learning data for the individual, the conceptual learning data associating the plurality of conceptual information with the plurality of corresponding neuronal stimulation signals (500, 600, 700) and the corresponding sensory percepts.

2. System (300, 140) according to claim 1, wherein the means for selecting and transmitting (320, 340, 350) the at least one neuronal stimulation signal (500, 600, 700) comprises at least one of: a digital signal processor (340); a digital to analog converter (220); a radio frequency transmitter (350, 310); a radio frequency receiver (142, 210); an analog and / or digital signal amplifier (230); radio frequency mixing circuitry (210, 350); low-pass, high-pass and / or bandpass circuitry (210, 350); wireless communication circuitry (210, 350); and impedance matching circuitry (210, 350).

3. System (300, 140) according to any of the preceding claims 1 or 2, wherein the stored relations (332) between the sensory percepts and the corresponding neuronal stimulation signals (500, 600, 700) are based at least in part on one or more of: spatial information for the at least one afferent axon (120); spatial information for the at least one neuronal stimulation means (130); neuronal connectivity information for the at least one afferent axon (120); an electric field distribution associated with the at least one neuronal stimulation means (130); functional neuroimaging data for the individual; diffusion tensor imaging data for the individual; neuroanatomical reference data being relevant for the individual; cortical excitation data for the individual; perceptual and / or conceptual learning data for the individual; sensory perception data for the individual; behavioral data based at least in part on subjective experiences of the individual; an optimization procedure for maximizing the number of sensory percepts that can be perceived, preferably simultaneously, by the individual when the corresponding neuronal stimulation signal (500, 600, 700) is transmitted to the at least one neuronal stimulation means (120) of the individual.

4. System (300, 140) according to the preceding claim 3, wherein the perceptual and / or conceptual learning data is generated based at least in part on one or more of: the individual participating in a perceptional and / or conceptual learning procedure (1000); the individual being analyzed by a non-invasive functional neuroimaging device, preferably functional magnetic resonance imaging or electrophysiological recording device or an invasive electrophysiological recording device while receiving the at least one neuronal stimulation signal.

5. System (300, 140) according to any of the preceding claims, wherein the conceptual information (510, 610, 710) comprises at least one of: a letter, a number, a color, a direction in space, a word, a sentence, an object, an identity of a person or animal, an instruction for a motor response of the individual, a position, a bio- or neurofeedback signal, a shape, an image or icon, a warning, an association, a degree of similarity, a salience signal, a rhythm, start or stop commands or information, touch information, surface texture information, pressure information, and / or an electro-magnetic field strength indication.

6. System (300, 140) according to any of the preceding claims, wherein the at least one neuronal stimulation means (130) comprises at least one stimulation electrode (130), preferably comprising a plurality of independently controllable electric stimulation contacts (132, 134, 136, 138) or wherein the at least one neuronal stimulation means (130) comprises at least two independently controllable stimulation electrodes (130), each preferably comprising a plurality of independently controllable electric contacts (132, 134, 136, 138).

7. System (300, 140) according to any of the preceding claims 5 or 6, wherein the at least one stimulation electrode is provided by at least a portion (1440) of a neuromodulation electrode (130) implanted for a therapeutic purpose (1430) independent from the stimulation.

8. System (300, 140) according to any of the preceding claims, wherein the at least one afferent axon (120) is a sensory afferent axon of the central nervous systems such as a thalamocortical axon.

9. System (300, 140) according to any of the preceding claims 1 to 8, wherein the at least one sensory neuron is located in at least one of: a somatosensory cortex area; an auditory cortex area; a visual cortex area; an olfactory cortex area; a gustatory cortex area; a somatosensory association cortex area; and a proprioception cortex area.

10. System according to any of the preceding claims wherein the selected neuronal stimulation signal (600') is adapted to provide a movement cue for the individual (100').

11. System according to claim 10, wherein the stored specific relations are based at least in part on movement cue learning data for the individual (100'), the learning data associating the plurality of movement cues with the plurality of corresponding neuronal stimulation signals (600').

12. System according to any of the preceding claims 10 or 11, wherein the neuronal stimulation signal (600') comprises a signal or a pulse train signal designed to be perceived by the individual (100') as periodic and wherein the means for transmitting (110', 412', 420') is further configured to control a frequency, a pulse width, a pulse shape and / or an amplitude of the neuronal stimulation signal (600') to control the movement speed, pace regularity and/or balance of the individual (100').

13. Computer program comprising instructions for implementing the system of any of the preceding claims, when being executed by a processing means and a transceiver means of a neuronal stimulation apparatus.

## Patentansprüche

1. System (300, 140) zum Übermitteln von konzeptioneller Information (510, 610, 710) an ein Individuum, umfassend:
Datenspeichermittel (330), die für das Individuum spezifische Beziehungen (332) zwischen einer Mehrzahl sensorischer Wahrnehmungen, die jeweils mit entsprechender konzeptioneller Information (510, 610, 710) assoziiert sind, und einer Mehrzahl entsprechender neuronaler Stimulationssignale (500, 600, 700) speichern;
wobei die gespeicherten Beziehungen jede sensorische Wahrnehmung, die durch das entsprechende neuronale Stimulationssignal hervorgerufen wird, mit der entsprechenden konzeptionellen Information verknüpfen;
Mittel zum Auswählen (320) mindestens eines neuronalen Stimulationssignals (500, 600, 700), das auf mindestens ein afferentes Axon (120) anzuwenden ist, das auf mindestens ein sensorisches Neuron in einem sensorischen Cortexbereich (100, 110, 112) des Individuums abzielt, wobei das Mittel zum Auswählen (310) des mindestens einen neuronalen Stimulationssignals (500, 600, 700) Mittel zum Zugreifen auf die Datenspeichermittel (330) umfassen;
wobei das ausgewählte mindestens eine neuronale Stimulationssignal (500, 600, 700) der konzeptionellen Information (510, 610, 710) entspricht, die an das Individuum zu übermitteln ist; und
Mittel zum Übertragen (340, 350, 310) des ausgewählten mindestens einen neuronalen Stimulationssignals (500, 600, 700) an ein neuronales Stimulationsmittel (130) des Individuums,
wobei das ausgewählte mindestens eine neuronale Stimulationssignal (500, 600, 700) angepasst ist, um eine Sequenz von Aktionspotentialen in dem mindestens einen afferenten Axon (120) hervorzurufen und dadurch eine entsprechende sensorische Wahrnehmung im abgezielten sensorischen Cortexbereich (100, 110, 112) des Individuums hervorzurufen, um die konzeptionelle Information, die mit der ausgewählten sensorischen Wahrnehmung verknüpft ist, an das Individuum zu übermitteln,
wobei die gespeicherten Beziehungen zumindest teilweise auf konzeptionellen Lerndaten für das Individuum basieren, wobei die konzeptionellen Lerndaten die Mehrzahl konzeptioneller Information mit der Mehrzahl entsprechender neuronaler Stimulationssignale (500, 600, 700) und den entsprechenden sensorischen Wahrnehmungen verknüpfen.

2. System (300, 140) nach Anspruch 1, wobei das Mittel zum Auswählen und Übertragen (320, 340, 350) des mindestens einen neuronalen Stimulationssignals (500, 600, 700) mindestens eines umfasst von: einem digitalen Signalprozessor (340); einem Digital-Analog-Wandler (220); einem Hochfrequenzsender (350, 310); einem Hochfrequenzempfänger (142, 210); einem analogen und/oder digitalen Signalverstärker (230); einer Hochfrequenz-Mischschaltung (210, 350); einem Tiefpass-, Hochpass- und/oder Band- -Pass-Schaltung (210, 350); einer drahtlosen Kommunikationsschaltung (210, 350); und einer Impedanzanpassungsschaltung (210, 350).

3. System (300, 140) nach einem der vorhergehenden Ansprüche 1 oder 2, wobei die gespeicherten Beziehungen (332) zwischen den sensorischen Wahrnehmungen und den entsprechenden neuronalen Stimulationssignalen (500, 600, 700) zumindest teilweise auf einem oder mehreren basieren von:
räumlicher Information für das mindestens eine afferente Axon (120);
räumlicher Information für das mindestens eine neuronale Stimulationsmittel (130); neuronaler Konnektivitätsinformation für das mindestens eine afferente Axon (120); einer elektrischen Feldverteilung, die mit dem mindestens einen neuronalen Stimulationsmittel (130) verknüpft ist; funktioneller Neurobildgebungsdaten für das Individuum; Diffusionstensor-Bildgebungsdaten für das Individuum; neuroanatomischer Referenzdaten, die für das Individuum relevant sind; kortikaler Anregungsdaten für das Individuum; Wahrnehmungs- und/oder konzeptioneller Lerndaten für das Individuum; sensorischer Wahrnehmungsdaten für das Individuum; Verhaltensdaten, die zumindest teilweise auf subjektiven Erfahrungen des Individuums basieren; einem Optimierungsverfahren zum Maximieren der Anzahl sensorischer Wahrnehmungen, die durch das Individuum, vorzugsweise gleichzeitig, wahrgenommen werden können, wenn das entsprechende neuronale Stimulationssignal (500, 600, 700) an das mindestens eine neuronale Stimulationsmittel (120) des Individuums übertragen wird.

4. System (300, 140) nach dem vorhergehenden Anspruch 3, wobei die Wahrnehmungs- und/oder konzeptionellen Lerndaten zumindest teilweise basierend auf einem oder mehreren von Folgendem erzeugt werden: dem Individuum, das an einer Wahrnehmungs- und/oder konzeptionellen Lernprozedur (1000) teilnimmt; dem Individuum, das durch eine nichtinvasive funktionelle Neurobildgebungsvorrichtung, vorzugsweise eine funktionelle Magnetresonanzbildgebungs- oder elektrophysiologische Aufzeichnungsvorrichtung oder eine invasive elektrophysiologische Aufzeichnungsvorrichtung analysiert wird, während es das mindestens eine neuronale Stimulationssignal empfängt.

5. System (300, 140) nach einem der vorhergehenden Ansprüche, wobei die konzeptionelle Information (510, 610, 710) mindestens eines von Folgendem umfasst: einen Buchstaben, eine Zahl, eine Farbe, eine Richtung im Raum, ein Wort, einen Satz, ein Objekt, eine Identität einer Person oder eines Tieres, eine Anweisung für eine motorische Reaktion des Individuums, eine Position, ein Bio- oder Neurofeedbacksignal, eine Form, ein Bild oder ein Symbol, eine Warnung, eine Assoziation, einen Ähnlichkeitsgrad, ein Salienzsignal, einen Rhythmus, Start- oder Stoppbefehle oder -information, Berührungsinformation, Oberflächentexturinformation, Druckinformation und/oder eine elektromagnetische Feldstärkeanzeige.

6. System (300, 140) nach einem der vorhergehenden Ansprüche, wobei das mindestens eine neuronale Stimulationsmittel (130) mindestens eine Stimulationselektrode (130) umfasst, die vorzugsweise eine Mehrzahl von unabhängig steuerbaren elektrischen Stimulationskontakten (132, 134, 136, 138) umfasst, oder wobei das mindestens eine neuronale Stimulationsmittel (130) mindestens zwei unabhängig steuerbare Stimulationselektroden (130) umfasst, die jeweils vorzugsweise eine Mehrzahl von unabhängig steuerbaren elektrischen Kontakten (132, 134, 136, 138) umfassen.

7. System (300, 140) nach einem der vorhergehenden Ansprüche 5 oder 6, wobei die mindestens eine Stimulationselektrode durch mindestens einen Abschnitt (1440) einer Neuromodulationselektrode (130) bereitgestellt wird, die für einen therapeutischen Zweck (1430) unabhängig von der Stimulation implantiert ist.

8. System (300, 140) nach einem der vorhergehenden Ansprüche, wobei das mindestens eine afferente Axon (120) ein sensorisches afferentes Axon des zentralen Nervensystems ist, wie etwa ein thalamokortikales Axon.

9. System (300, 140) nach einem der vorhergehenden Ansprüche 1 bis 8, wobei sich das mindestens eine sensorische Neuron in mindestens einem befindet von:
einem somatosensorischen Cortexbereich; einem auditorischen Cortexbereich;
einem visuellen Cortexbereich; einem olfaktorischen Cortexbereich; einem gustatorischen Cortexbereich; einem somatosensorischen Assoziationscortexbereich; und einem Propriozeptionscortexbereich.

10. System nach einem der vorhergehenden Ansprüche, wobei das ausgewählte neuronale Stimulationssignal (600') angepasst ist, um einen Bewegungshinweis für das Individuum (100') bereitzustellen.

11. System nach Anspruch 10, wobei die gespeicherten spezifischen Beziehungen zumindest teilweise auf Bewegungshinweis-Lerndaten für das Individuum (100') basieren, wobei die Lerndaten die Mehrzahl Bewegungshinweise mit der Mehrzahl entsprechender neuronaler Stimulationssignale (600') verknüpfen.

12. System nach einem der vorhergehenden Ansprüche 10 oder 11, wobei das neuronale Stimulationssignal (600') ein Signal oder ein Impulsfolgesignal umfasst, das ausgelegt ist, um von dem Individuum (100') als periodisch wahrgenommen zu werden, und wobei das Mittel zum Übertragen (110', 412', 420') ferner konfiguriert ist, um eine Frequenz, eine Impulsbreite, eine Impulsform und/oder eine Amplitude des neuronalen Stimulationssignals (600') zu steuern, um die Bewegungsgeschwindigkeit, die Schrittregelmäßigkeit und/oder das Gleichgewicht des Individuums (100') zu steuern.

13. Computerprogramm, umfassend Anweisungen zum Implementieren des Systems nach einem der vorhergehenden Ansprüche, wenn es von einem Verarbeitungsmittel und einem Sendeempfängermittel einer neuronalen Stimulationsvorrichtung ausgeführt wird.

## Revendications

1. Système (300, 400) pour la communication d'informations conceptuelles (510, 610, 710) à un individu, comprenant :
un moyen de stockage de données (330) stockant des relations (332), propres à l'individu, entre une pluralité de perceptions sensorielles, associées chacune à une information conceptuelle correspondante (510, 610, 710), et une pluralité de signaux de stimulation neuronale correspondants (500, 600, 700) ;
dans lequel les relations stockées relient chaque perception sensorielle, induite par le signal de stimulation neuronale correspondant, à l'information conceptuelle correspondante ;
un moyen de sélection (320) d'au moins un signal de stimulation neuronale (500, 600, 700) à appliquer à au moins un axone afférent (120) ciblant au moins un neurone sensoriel dans une aire sensorielle du cortex (100, 110, 120) de l'individu, le moyen de sélection (310) de l'au moins un signal de stimulation neuronale (500, 600, 700) comprenant un moyen pour accéder au moyen de stockage de données (330) ;
dans lequel l'au moins un signal de stimulation neuronale sélectionné (500, 600, 700) correspond à l'information conceptuelle (510, 610, 710) à communiquer à l'individu ; et
un moyen de transmission (340, 350, 310) de l'au moins un signal de stimulation neuronale sélectionné (500, 600, 700) à un moyen de stimulation neuronale (130) de l'individu,
dans lequel l'au moins un signal de stimulation neuronale sélectionné (500, 600, 700) est apte à induire une séquence de potentiels d'action dans l'au moins un axone afférent (120), et induire ainsi une perception sensorielle correspondante dans la zone sensorielle ciblée du cortex (100, 110, 112) de l'individu pour communiquer à l'individu l'information conceptuelle associée à la perception sensorielle sélectionnée,
dans lequel les relations stockées sont au moins en partie basées sur des données d'apprentissage conceptuel pour l'individu, les données d'apprentissage conceptuel associant la pluralité d'informations conceptuelles à la pluralité de signaux de stimulation neuronale correspondants (500, 600, 700) et aux perceptions sensorielles correspondantes.

2. Système (300, 140) selon la revendication 1, dans lequel le moyen de sélection et de transmission (320, 340, 350) de l'au moins un signal de stimulation neuronale (500, 600, 700) comprend au moins l'un parmi : un processeur de signaux numériques (340) ; un convertisseur numérique vers analogique (220) ; un émetteur radiofréquence (350, 310) ; un récepteur radiofréquence (142, 210) ; un amplificateur de signal analogique et/ou numérique (230) ; une circuiterie mélangeuse radiofréquence (210, 350) ; une circuiterie passe-bas, passe-haut et/ou passe-bande ; une circuiterie de communication sans fil (210, 350) ; et une circuiterie d'adaptation d'impédance (210, 350).

3. Système (300, 140) selon l'une des revendications 1 ou 2 précédentes, dans lequel les relations stockées (332) entre les perceptions sensorielles et les signaux de stimulation neuronale correspondants (500, 600, 700) sont au moins en partie basées sur une ou plusieurs parmi : une information spatiale pour l'au moins un axone afférent (120) ; une information spatiale pour l'au moins un moyen de stimulation neuronale (130) ; une information de connectivité neuronale pour l'au moins un axone afférent (120) ; une distribution de champ électrique associée à l'au moins un moyen de stimulation neuronale (130) ; des données de neuro-imagerie fonctionnelle pour l'individu ; des données d'imagerie de tenseur de diffusion pour l'individu ; des données de référence neuro-anatomiques pertinentes pour l'individu ; des données d'excitation corticale pour l'individu ; des données d'apprentissage perceptuel et/ou conceptuel pour l'individu ; des données de perception sensorielle pour l'individu ; des données comportementales au moins en partie basées sur des expériences subjectives de l'individu ; une procédure d'optimisation pour la maximisation du nombre de perceptions sensorielles qui peuvent être perçues, de préférence simultanément, par l'individu lorsque le signal de stimulation neuronale correspondant (500, 600, 700) est transmis à l'au moins un moyen de stimulation neuronale (120) de l'individu.

4. Système (300, 140) selon la revendication 3 précédente, dans lequel les données d'apprentissage perceptuel et/ou conceptuel sont générées au moins en partie sur la base d'une ou plusieurs parmi : la participation de l'individu à une procédure d'apprentissage perceptuel et/ou conceptuel (1000) ; l'analyse de l'individu par un dispositif non invasif de neuro-imagerie fonctionnelle, de préférence un dispositif d'imagerie fonctionnelle par résonance magnétique ou d'enregistrement électrophysiologique, ou un dispositif invasif d'enregistrement électrophysiologique lors de la réception de l'au moins un signal de stimulation neuronale.

5. Système (300, 1140) selon l'une des revendications précédentes, dans lequel l'information conceptuelle (510, 610, 710) comprend au moins l'un parmi : une lettre, un nombre, une couleur, une direction dans l'espace, un mot, une phrase, un objet, une identité d'une personne ou d'un animal, une instruction pour une réponse motrice de l'individu, une position, un signal de bio- ou neuro-feedback, une forme, une image ou une icône, une alerte, une association, un degré de similarité, un signal de saillance, un rythme, des commandes ou une information de départ ou d'arrêt, une information de toucher, une information de texture de surface, une information de pression, et/ou une indication d'intensité de champ électromagnétique.

6. Système (300, 140) selon l'une des revendications précédentes, dans lequel l'au moins un moyen de stimulation neuronale (130) comprend au moins une électrode de stimulation (130), de préférence comprenant une pluralité de contacts de stimulation électrique contrôlables indépendamment (132, 134, 136, 138), ou dans lequel l'au moins un moyen de stimulation neuronale (130) comprend au moins deux électrodes de stimulation contrôlables indépendamment (130), de préférence comprenant chacune une pluralité de contacts électriques contrôlables indépendamment (132, 134, 136, 138).

7. Système (300, 140) selon l'une des revendications 5 ou 6 précédentes, dans lequel l'au moins une électrode de stimulation est obtenue par au moins une partie (1440) d'une électrode neuromodulation (130) implantée à des fins thérapeutiques (1430) indépendamment de la stimulation.

8. Système (300, 140) selon l'une des revendications précédentes, dans lequel l'au moins un axone afférent (120) est un axone afférent sensoriel du système nerveux central, tel qu'un axone thalamo-cortical.

9. Système (300, 140) selon l'une des revendications 1 à 8 précédentes, dans lequel l'au moins un neurone sensoriel est situé dans au moins l'une d'entre : une aire somato-sensorielle du cortex, une aire de l'audition du cortex ; une aire de la vision du cortex ; une aire de l'olfaction du cortex ; une aire du goût du cortex ; une aire d'association somato-sensorielle du cortex ; et une aire de proprioception du cortex.

10. Système selon l'une des revendications précédentes, dans lequel le signal de stimulation neuronale sélectionné (600') est apte à produire une perception d'un mouvement pour l'individu (100').

11. Système selon la revendication 10, dans lequel les relations propres stockées sont au moins en partie basées sur des données d'apprentissage de perception de mouvement pour l'individu (100'), les données d'apprentissage associant la pluralité de perceptions d'un mouvement à la pluralité de signaux de stimulation neuronale correspondants (600').

12. Système selon l'une des revendications 10 ou 11 précédentes, dans lequel le signal de stimulation neuronale (600') comprend un signal, ou un signal de train d'impulsions, conçu pour être perçu par l'individu (100') comme étant périodique, et dans lequel le moyen de transmission (110', 412', 420') est en outre configuré pour contrôler une fréquence, une largeur d'impulsion, une forme d'impulsion et/ou une amplitude du signal de stimulation neuronale (600') pour contrôler la vitesse d'un mouvement, la régularité d'une cadence et/ou l'équilibre de l'individu (100').

13. Programme informatique comprenant des instructions pour implémenter le système de l'une des revendications précédentes, lorsqu'elles sont exécutées par un moyen de traitement et par un moyen émetteur-récepteur d'un appareil de stimulation neuronale.
